(19) Europäisches Patentamt
European Patent Office
Office européen des brevets

(11) Veröffentlichungsnummer: **0 021 169**
**B1**

(12) # EUROPÄISCHE PATENTSCHRIFT

(45) Veröffentlichungstag der Patentschrift: **07.03.84**

(51) Int. Cl.³: **C 07 C 103/52,** A 61 K 37/26

(21) Anmeldenummer: **80103128.7**

(22) Anmeldetag: **04.06.80**

(54) **Verfahren zur selektiven Bildung von Disulfidbrücken in Polypeptiden und die dabei erhaltenen Produkte als Wirkstoffe enthaltende Arzneimittel.**

(30) Priorität: **12.06.79 DE 2923787**

(43) Veröffentlichungstag der Anmeldung: **07.01.81 Patentblatt 81/1**

(45) Bekanntmachung des Hinweises auf die Patenterteilung: **07.03.84 Patentblatt 84/10**

(84) Benannte Vertragsstaaten: **BE CH FR GB IT LI SE**

(56) Entgegenhaltungen:
**FR - A - 2 230 622**

**CHEMICAL ABSTRACTS, Band 92, Nr. 3, 21. Januar 1980, Zusammenfassung Nr. 22812t, Seite 716, Columbus, Ohio, US, H. KUNZEK et al.: "Selectively S-protected cysteine peptides. III. Synthesis of a (Ala12)-sheep insulin A-chain octapeptide with 6-11-disulfide ring"**
**JOURNAL OF THE CHEMICAL SOCIETY, Chem. Comm., 1976, LONDON, GB, S. MATSUURA ET AL.: "PYRIDINIUM POLYhydrogen fluoride, a deprotecting reagent in peptide chemistry", Seiten 451-452**
**HOUBEN WEYL: "Methoden der Organischen Chemie,", Thieme Verlag, Band XV/1, 1974, Stuttgart, DE, "Synthese von Peptiden", Seite 743**

(73) Patentinhaber: **Birr, Christian, Dr., Werderstrasse 35, D-6900 Heidelberg (DE)**

(72) Erfinder: **Birr, Christian, Dr., Werderstrasse 35, D-6900 Heidelberg (DE)**

(74) Vertreter: **Weickmann, Heinrich, Dipl.-Ing. et al, Patentanwälte Dipl.-Ing. H.Weickmann Dipl.-Phys.Dr. K. Fincke Dipl.-Ing. F.A. Weickmann Dipl.-Chem. B. Huber Dr.-Ing. H. Liska Dipl.-Phys.Dr. J. Prechtel Möhlstrasse 22, D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur selektiven Bildung von drei Disulfidbrücken in Polypeptiden und die dabei erhaltenen Produkte als Wirkstoffe enthaltende Arzneimittel

Gegenstand der Erfindung ist ein Verfahren zur selektiven Bildung von drei Disulfidbrücken, gegebenenfalls in Gegenwart von einer oder mehreren schon bestehenden weiteren Disulfidbrücken, in synthetischen und/oder natürlichen Polypeptiden, namentlich ein Verfahren zur Herstellung von Insulinderivaten, sowie Arzneimittel, die die in dieser Weise erhaltenen Produkte als Wirkstoffe enthalten.

In letzter Zeit hat die biologische Insulin-Synthese durch Escherichia coli-Mutanten erhebliches Aufsehen erregt (siehe D.V. Doeddel, D.G. Kleid, F. Bolivar, H.L. Heynecher, D.G. Yansura, R. Crea, T. Hirose, A. Krazewski, K. Itakura und A.D. Riggs, Proc. Natl. Acad. Sci. USA 76 (im Druck)). Wegen der Gefahr der Infizierung des Menschen mit Insulin produzierenden Bakterien werden dabei die Insulin A- und B-Ketten in verschiedenen Kulturen getrennt voneinander aufgebaut und als freie Polypetidketten gewonnen. Sie werden anschliessend durch statistische Disulfid-Oxidation zu dem Insulinmolekül verbrückt, wie dies auch bei den früheren Totalsynthesen des Insulins der Fall ist (R.E. Humbel, H.R. Bosshard, H. Zahn in D.F. Steiner und N. Freinkel "Handbook of Physiology", Vol. 1, Williams & Wilkins, Baltimor (1972), Seite 111 ff). Bei dieser statistischen Vereinigung der beiden Ketten entsteht aber Insulin nur als ein Produkt unter vielen möglichen (3 Monomere Bis-Disulfide der A-Kette, 1 Disulfid der B-Kette, 12 isomere Insuline und viele Oligomere und Polymere einzelner und beider Ketten), so dass es bislang nicht gelang, die A- und die B-Kette durch statistische Disulfidsynthese in höheren Ausbeuten als 10 bis 20% zum Insulin zu vereinigen. Auch Rekombinationsversuche mit natürlichen A- und B-Ketten im Verhältnis 1 : 1 ergaben nur Insulinausbeuten von ca. 10%.

B. Kamber et al. berichteten bereits über eine Totalsynthese von Humaninsulin, bei der die drei Disulfidbrücken erstmals gezielt auf verschiedenen Stufen eines fragmentartigen Aufbaus gebildet wurden (Helvetica Chimica Acta, Vol 57, Fasc. 8 (1974), Seiten 2617–2621 und Vol. 60, Fasc. 1 (1977), Seiten 27–37 und die dort genannten Literaturstellen). Auch in diesem Fall werden die interchenaren Disulfidbrücken der Peptidketten A und B des Insulins nicht erst durch Verbindungen dieser kompletten beiden Ketten gebildet, sondern auf Vorstufen der Synthese.

Neben der Grösse und Komplexität der das Hormon Insulin bildenden Peptidketten A und B ist das Problem der Insulin-Synthese also vor allen dadurch gekennzeichnet, dass die das Molekül intra- und interchenar verbindenden drei Disulfidbrücken gezielt und nicht statistisch geschlossen werden sollte, um die Ausbeuten an Insulin zu verbessern. Dabei soll möglichst von insbesondere biologisch gebildeten A- und B-Ketten des Insulins ausgegangen werden können.

Humaninsulin entspricht der folgenden Formel I

Gly–Ile–Val–Glu–Gln–Cys–Cys–Thr–Ser–Ile–Cys–Ser–Leu–Tyr–Gln–Leu–Glu–Asn–Tyr–Cys–Asn
1 2 3 4 5 6 7 8 9 10 11 12 13 14 15 16 17 18 19 20 21

Phe–Val–Asn–Gln–His–Leu–Cys–Gly–Ser–His–Leu–Val–Glu–Ala–Leu–Tyr–Leu–Val–Cys–Gly–Glu
1 2 3 4 5 6 7 8 9 10 11 12 13 14 15 16 17 18 19 20 21

Arg–Gly–Phe–Phe–Tyr–Thr–Pro–Lys–Thr
22 23 24 25 26 27 28 29 30

I

In der obigen Formel I entspricht die obere Aminosäuresequenz (1 bis 21) der A-Kette, während die untere Aminosäuresequenz (1 bis 30) die B-Kette des Humaninsulins darstellt.

Die FR-A-2 230 622 beschreibt ein Verfahren zur Bildung von mehreren Disulfidbrücken in Polypeptiden, gemäss dem als SH-Schutzgruppen Aralkylgruppen bzw. Acylaminomethylgruppen verwendet werden, die vom gleichen $J_2$-Reagens abgespalten und gleichzeitig zu den gewünschten Disulfidbrücken oxidiert werden. Nach dieser Verfahrensweise ist es aber nicht möglich, die selektive Ausbildung von drei Disulfidbrücken zu erreichen, so dass es bereits im Verlaufe der Peptidsynthese erforderlich ist, die Disulfidbrücken zu schliessen. Dies erschwert die Synthese ausserordentlich, da mehrere Peptidketten vorliegen, an denen weitere Reaktionen beim Aufbau der Peptidkette ablaufen können.

Aus J.C.S. Chem. Comm. (1976), Seiten 451–452 ist es bereits bekannt, dass man Pyridinium-polyhydrogenfluorid als Mittel zur Abspaltung von Schutzgruppen in der Peptidchemie einsetzen kann, während anderseits aus Houben-Weyl "Methoden der organischen Chemie", Thieme Verlag, Band XV/1 (1974), Seite 743 bekannt ist, dass man die S-4-Methoxybenzylschutzgruppe in Gegenwart von Anisol auch mit Fluorwasserstoff abspalten kann.

Die dem Anmeldungsgegenstand zugrunde liegende Aufgabe ist nun darin zu sehen, ein Verfahren anzugeben, mit dem es gelingt, selektiv drei Disulfidbrücken in natürlichen und/oder synthetischen Polypeptiden zu bilden, und dies gegebenenfalls in Gegenwart von einer oder mehreren bereits bestehenden Disulfidbrücken, wobei dieses Verfahren insbesondere für die Herstellung von synthetischem oder halbsynthetischem Humaninsulin durch Kombination einer synthetischen A-Kette des Insulins mit einer synthetischen oder natürlichen B-Kette des Insulins geeignet sein soll.

Es wurde nunmehr gefunden, dass es gelingt, Disulfidbrücken selektiv auszubilden, wenn man bestimmte Aminosäure-Schutzgruppen anwendet und diese in bestimmter Weise unter gleichzeitiger Disulfidbrückenbildung wieder abspaltet. So hat es sich überraschenderweise gezeigt, dass, wenn man eine oder beide SH-Gruppen, die in eine erste Disulfidbrücke umgewandelt werden sollen, mit einer p-Methoxybenzylschutzgruppe und eine oder beide bzw. weitere SH-Gruppen, die in eine zweite oder weitere Disulfidbrücken umgewandelt werden sollen, mit einer Acetamidomethylschutzgruppe maskiert und man dann die p-Methoxybenzylschutzgruppe mit Pyridinium-polyhydrogenfluorid (HF/Pyridin) in Gegenwart von Anisol abspaltet und anschliessend mit Jod in saurer Lösung behandelt, wodurch zunächst die erste Disulfidbrücke geschlossen wird, wonach die Acetamidomethylschutzgruppen abgespalten und die in dieser Weise freigesetzten SH-Gruppen zu der zweiten bzw. weiteren Disulfidbrücke oxidiert werden.

Gegenstand der Erfindung ist daher ein Verfahren zur selektiven Bildung von drei Disulfidbrücken, gegebenenfalls in Gegenwart von einer oder mehreren schon bestehenden weiteren Disulfidbrücken, in synthetischen und/oder natürlichen Polypeptiden, das dadurch gekennzeichnet ist, dass man wenigstens eine der beiden in eine erste Disulfidbrücke zu überführenden SH-Gruppen mit einer tert.-Butylmercaptoschutzgruppe, wenigstens eine der beiden in eine zweite Disulfidbrücke zu überführenden SH-Gruppen mit einer p-Methoxybenzylschutzgruppe und wenigstens eine der beiden in eine dritte Disulfidbrücke zu überführenden SH-Gruppen mit einer Acetamidomethylschutzgruppe maskiert, dann zuerst die tert.-Butylmercaptoschutzgruppe bzw. -gruppen mit Tributylphosphan unter Stickstoff abspaltet, mit Luft oxidiert, dann die p-Methoxybenzylschutzgruppe bzw. -gruppen mit Pyridinium-polyhydrogenfluorid (HF-Pyridin) in Gegenwart von Anisol abspaltet und anschliessend mit Jod in saurer Lösung behandelt.

Einer bevorzugten Ausführungsform der Erfindung zufolge wird das Verfahren auf die Herstellung von aktivem Humaninsulin angewandt, wozu man eine synthetische Insulin-A-Kette, die in der Position $A^7$ eine Acetamidomethylschutzgruppe und in der Position $A^{20}$ eine p-Methoxybenzylschutzgruppe aufweist, mit einer äquimolaren Menge synthetischer oder natürlicher, reduzierter Insulin-B-Kette umsetzt.

Mit Vorteil verwendet man dabei eine Insulin-B-Kette, die in der Position $B^7$ eine Acetamidomethylschutzgruppe und in der Position $B^{19}$ eine p-Methoxybenzylschutzgruppe aufweist, da in dieser Weise die Bildung von Isomeren des Insulins vollständig unterdrückt werden kann.

In dieser Weise gelingt es, selektiv die vier zu verbindenden SH-Gruppen mit dem jeweils richtigen Reaktionspartner unter Bildung von zwei Disulfidbrücken zu verknüpfen, ohne dass dabei schon existierende Disulfidbindungen gefährdet werden, wie der bereits in der A-Kette vorliegende intrachenare kleine Disulfidring [$A^6$–$A^{11}$].

Bei der bevorzugten erfindungsgemässen Verfahrensweise zur Herstellung von halbsynthetischem Humaninsulin geht man von einer vollsynthetischen A-Kette aus, die vier SH-Gruppen aufweist, von denen zwei, die zum dem kleinen intrachenaren Disulfidring zu schliessen sind, mit tert.-Butylmercaptoschutzgruppen maskiert sind, während die beiden übrigen SH-Gruppen eine p-Methoxybenzylgruppe bzw. eine Acetamidomethylgruppe aufweisen, nämlich in den Positionen $A^{20}$ bzw. $A^7$. Dabei werden zunächst selektiv die tert.-Butylmercaptogruppen durch Behandeln mit Tributylphosphan unter Stickstoff abgespalten, worauf durch selektive Oxidation mit Luft die Disulfidbrücke unter Bildung des kleinen Rings intrachenar geschlossen wird. Bei dieser Behandlungsweise bleiben die Acetamidomethylschutzgruppe und die p-Methoxybenzylschutzgruppe unbeeinflusst. Anschliessend wird nun nach der erfindungsgemässen Verfahrensweise durch Behandlung mit Pyridinium-polyhydrogenfluorid (HF/Pyridin) die p-Methoxybenzylschutzgruppe abgespalten, wobei die Acetamidomethylschutzgruppe erhalten bleibt. Anschliessend wird in Gegenwart einer natürlichen B-Kette des Insulins, in der die beiden SH-Gruppen ungeschützt vorliegen, mit Jod in 30%iger Essigsäure behandelt. Hierbei werden nacheinander zunächst die zweite Disulfidbrücke geknüpft ($A^{20}$–$B^{19}$), dann die Acetamidomethylschutzgruppe abgespalten und die dabei freigesetzte SH-Gruppe mit der weiteren freien noch verbliebenen SH-Gruppe der B-Kette zur dritten Disulfidbrücke ($A^7$–$B^7$) oxidiert. Hierbei hat sich gezeigt, dass überraschenderweise ca. 30% der eingesetzten Menge freier B-Kette selektiv mit der richtigen SH-Gruppe der B-Kette reagiert.

Um die Disulfid-Verbrückungsreaktion jedoch noch genauer zu steuern, ist es zweifellos möglich, von einer natürlichen oder synthetischen Insulin-B-Kette auszugehen, die in der Position $B^7$ eine Acetamidomethylschutzgruppe und in der Position $B^{19}$ eine p-Methoxybenzylschutzgruppe aufweist, so dass die entsprechenden SH-Gruppen der A- bzw. B-Kette des Insulins ensprechend maskiert sind und dann bei der Durchführung der

beanspruchten Verfahrensweise in der angegebenen Reaktionsfolge jeweils nur eine SH-Gruppe in jeder der beiden Ketten für die Reaktion zur Verfügung steht.

Nach diesem Prinzip des erfindungsgemässen Verfahrens ist es natürlich auch möglich, innerhalb einer einzigen Kette zwei verschiedene Ringe über Disulfidbrücken zu bilden bzw. zwei oder mehrere Disulfidbrücken in einer oder mehreren Ketten auszubilden. Auch in diesem Falle werden die miteinander zur Reaktion zu bringenden SH-Gruppen erfindungsgemäss mit gleichartigen Maskierungsgruppen geschützt.

Das erfindungsgemässe Verfahren ist von besonderer Bedeutung für die Synthese des Humaninsulins. Bei den verschiedenen Insulinen ist je nach Herkunft in der Regel die B-Kette, abgesehen vom C-Terminus, mit der B-Kette des Humaninsulins identisch, während die A-Ketten deutlich verschieden sind. Da der C-Terminus der B-Kette leicht ausgetauscht werden kann, genügt es, die A-Kette zu synthetisieren und eine B-Kette natürlichen Ursprungs nach Änderung des C-Terminus einzusetzen, um dann, ausgehend von diesen Ausgangsmaterialien, nach der erfindungsgemässen Verfahrensweise Humaninsulin zu bilden.

Das erfindungsgemässe Verfahren ist jedoch auch für die Herstellung von Insulinanalogen geeignet, wie sie beispielsweise in den nachstehenden schematischen Formeln II und VI verdeutlicht werden.

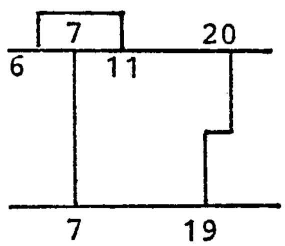


I

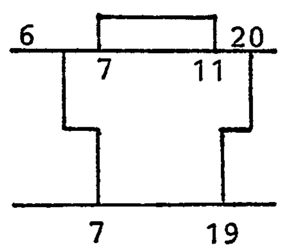


II

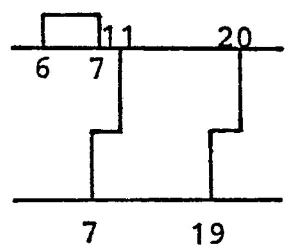


III

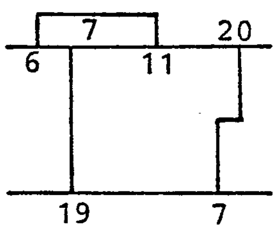


IV

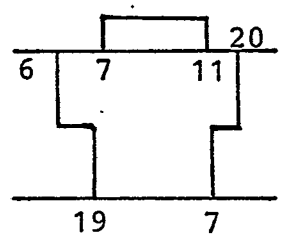


V

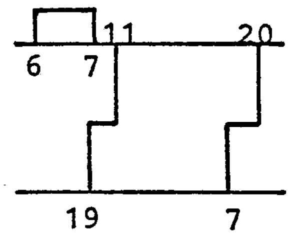


VI

Die obigen Formeln I bis IV geben in schematisierter Weise Insulinderivate wieder, die mit Hilfe des erfindungsgemässen Verfahrens zugänglich sind. So steht die allgemeinen Formel I für das natürliche Insulin, während die Formeln II und III Insulinderivate mit einem unnatürlichen intrachenaren Disulfidring wiedergeben, bei denen der Disulfidring in der $A^7$–$A^{11}$– bzw. in der $A^6$–$A^7$-Position vorliegt. Die Formeln IV bis VI verdeutlichen die antiparallelen Varianten der Verbindungen der Formeln I bis III, bei denen die Insulin-B-Kette antiparallel an die entsprechende Insulin-A-Kette gebunden ist.

Diese letzteren antiparallen Varianten des Insulins mit natürlicher oder unnatürlicher Anordnung der intrachenaren Disulfidbrücke sind von besonderer Bedeutung, da sie unter bestimmten pH-Bedingungen allmählich die natürliche Form der Verbrückung rekonstruieren, so dass sie langlebige, unwirksame, künstlich erzeugte Depotformen des Insulins darstellen, die sich unter physiologischen Bedingungen ganz langsam, wie es in der Diabetes-Therapie erwünscht ist, in das wirksame Insulin umwandeln.

Für die Herstellung des Insulinanalogen der Formel II ([$A^7$–$A^{11}$, $A^6$–$B^7$-Cystin]-Insulin) verwendet man als Ausgangsmaterial eine synthetische [$A^7$–$A^{11}$]-Insulin-A-Kette, die in der Position $A^6$ eine Acetamidomethylschutzgruppe und in der Position $A^{20}$ eine p-Methoxybenzylschutzgruppe aufweist.

Für die Herstellung des Insulinanalogen der obigen Formel III ([$A^6$–$A^7$, $A^{11}$–$B^7$-Cystin]-Insulin) verwendet man als Ausgangsmaterial eine [$A^6$–$A^7$]-Insulin-A-Kette, die in der Position $A^{11}$ eine Acetamidomethylschutzgruppe und in der Position $A^{20}$ eine p-Methoxybenzylschutzgruppe aufweist.

Für die Herstellung des antiparallelen Insulinanalogen der obigen Formel IV ([$A^6$–$A^{11}$, $A^7$–$B^{19}$, $A^{20}$–$B^7$-Cystin]-Insulin) verwendet man als Ausgangsmaterial eine natürliche oder synthetische [$A^6$–$A^{11}$]-Insulin-A-Kette, die in der Position $A^7$ bzw. $A^{20}$ eine Acetamidomethylschutzgruppe und in der Position $A^{20}$ bzw. $A^7$ eine p-Methoxybenzylschutzgruppe aufweist, die man mit einer natürlichen oder synthetischen Insulin B-Kette umsetzt, die in reduzierter Form vorliegt, oder bevorzugter

in der Position $B^{19}$ bzw. $B^7$ eine Acetamidomethylschutzgruppe und in der Position $B^7$ bzw. $B^{19}$ eine p-Methoxybenzylschutzgruppe aufweist.

Für die Herstellung des antiparallelen Insulinanalogen der obigen Formel V [$A^7$–$A^{11}$, $A^6$–$B^{19}$, $A^{20}$–$B^7$-Cystin]-Insulin) setzt man eine synthetische [$A^7$–$A^{11}$]-Insulin-A-Kette, die in der Position $A^6$ bzw. $A^{20}$ eine Acetamidomethylschutzgruppe und in der Position $A^{20}$ bzw. $A^6$ eine p-Methoxybenzylschutzgruppe aufweist, mit einer natürlichen oder synthetischen Insulin B-Kette um, die in reduzierter Form vorliegt oder bevorzugter in der Position $B^{19}$ bzw. $B^7$ eine Acetamidomethylschutzgruppe und in der Position $B^7$ bzw. $B^{19}$ eine p-Methoxybenzylschutzgruppe aufweist.

Zur Herstellung des antiparallelen Insulinanalogen der obigen Formel VI ([$A^6$–$A^7$, $A^{11}$–$B^{19}$, $A^{20}$–$B^7$-Cystin]-Insulin) setzt man eine synthetische [$A^6$–$A^7$]-Insulin-A-Kette, die in der Position $A^{11}$ bzw. $A^{20}$ eine Acetamidomethylschutzgruppe und in der Position $A^{20}$ bzw. $A^{11}$ eine p-Methoxybenzylschutzgruppe aufweist, mit einer natürlichen oder synthetischen Insulin B-Kette um, die in reduzierter Form vorliegt, oder bevorzugter in der Position $B^{19}$ bzw. $B^7$ eine Acetamidomethylschutzgruppe und in der Position $B^7$ bzw. $B^{19}$ eine p-Methoxybenzylschutzgruppe aufweist.

Gegenstand der Erfindung ist ferner ein Verfahren, das dadurch gekennzeichnet ist, dass man eine natürliche oder synthetische Insulin A-Kette mit dem intrachenaren Disulfidring in der $A^6$–$A^{11}$-Position, in der $A^7$–$A^{11}$-Position oder in der $A^6$–$A^7$-Position an einem polymeren Träger parallel oder antiparallel mit einer natürlichen oder synthetischen Insulin B-Kette, die in reduzierter Form vorliegt oder Acetamidomethylschutzgruppen bzw. p-Methoxybenzylschutzgruppen in den oben definierten Positionen aufweist, Disulfid-verbrückt. Hierbei erhält man eine besonders langlebige, unphysiologische Insulin-Depotform, die für therapeutische Anwendungszwecke von hohem Nutzen ist.

Bei der Durchführung des erfindungsgemässen Verfahrens bewirkt man die Disulfidbrückenbildung durch Oxidation mit Jod vorzugsweise in 30%iger wässriger Essigsäure.

Die Abspaltung der p-Methoxybenzylschutzgruppe bzw. -gruppen bewirkt man mit Vorteil in der Weise, dass man das Reagens Pyridinium-polyhydrogenfluorid (HF/Pyridin) als Lösungsmittel einsetzt.

Die Verwendung von Pyridinium-polyhydrogenfluorid (HF/Pyridin) als Mittel zur Abspaltung von Schutzgruppen in der Peptidchemie ist bereits bekannt (siehe J.C.S. Chem. Comm (1976) 451 und 452). Es war jedoch nicht zu erwarten, dass die Abspaltung der p-Methoxybenzylschutzgruppe in Gegenwart von Anisol als Kationenfänger besonders glatt gelingt und dies ohne Beeinträchtigung der noch vorhandenen Acetamidomethylschutzgruppe und ohne Beeinträchtigung eventuell bereits vorhandener Disulfidbrücken.

Im folgenden sei die Erfindung näher im Hinblick auf die Synthese von Rinderinsulin erläutert. Dabei sei zunächst die an sich bekannte Fragmentsynthese einer Insulin A-Kette mit selektiv abspaltbaren Schwefelschutzgruppen am polymeren Träger sowie die erfindungsgemässe Umsetzung dieser synthetischen A-Kette unter schrittweiser, oxidativer Disulfidverbrückung mit einer natürlichen B-Kette zu einem voll aktiven, kristallinen Rinderinsulin erläutert. Das hierbei angewandte Konzept ist eine Weiterentwicklung der Prinzipien, die zur Synthese des MCD-Peptids führten (C. Birr und M. Wengert-Müller, Angewandte Chemie 91 (1979) 156 und DE-OS 2 830 442).

Als Schwefelschutzgruppen werden hierbei am Cystein in den Positionen $A^6$ und $A^{11}$ das Disulfid mit tert.-Butylmercaptan (StBu bzw. SBu) in der Position $A^7$ die Acetamidomethylschutzgruppe (Acm bzw. ACM) und in der Position $A^{20}$ die p-Methoxybenzylschutzgruppe (Mbzl bzw. MBZL) verwendet.

Nachfolgend sei die Erfindung näher unter Bezugnahme auf die beigefügten Zeichnungen erläutert.

In den Zeichnungen zeigen:

Fig. 1 das gesamte Syntheseschema der Insulin A-Kette;

Fig. 2 das Syntheseschema des Fragments II der Insulin A-Kette;

Fig. 3 das Syntheseschema des Fragments IV der Insulin A-Kette;

Fig. 4 die Verknüpfung der Fragmente III und IV unter Bildung des kleinen intrachenaren Disulfidrings zur Insulin A-Kette und deren erfindungsgemässe Verknüpfung mit einer Rinderinsulin B-Kette und

Fig. 5 eine Mikrophotographie des Zinkkomplexes des erfindungsgemäss semisynthetisch hergestellten voll aktiven Rinderinsulins.

Die in den Fig. 1 bis 4 angegebenen Abkürzungen besitzen die folgenden Bedeutungen:

| | |
|---|---|
| DDZ bzw. Ddz | = α,α-Dimethyl-3,5-dimethoxy-benzyloxy-carbonylgruppe, |
| OME | = Methoxygruppe, |
| OBU bzw. $OBu^t$ | = tert.-Butoxygruppe, |
| OBZL bzw. OBzl | = Benzyloxygruppe, |
| SBU bzw. $SBu^t$ | = Butylmercaptogruppe, |
| ACM bzw. Acm | = Acetamidomethylgruppe, |
| BU bzw. $Bu^t$ | = Butylgruppe, |
| MBZL bzw. Mbzl | = p-Methoxybenzylgruppe. |

Über die Herstellung der einzelnen Fragmente I, II, III und IV wurde bereits von C. Birr im Jahre 1978 in Danzig berichtet.

Man bildet das Fragment I (1–3) aus Ddz-Aminosäuren in Lösung mit einer Ausbeute von 84%, nach einer Methode, die kürzlich publiziert wurde (C. Birr et al., Int. J. Peptide Protein Res 13 (1979) 287–295). Die Fragmente II (4–14) und IV (15–21), siehe die in den Fig. 2 und 3 dargestellten Schemata, werden an mit 0,5% Divinylbenzol vernetztem Polystyrolgel aufgebaut. Bezogen auf die Anfangsbeladung erzielt man Ausbeuten von 90% (Fragment II) bzw. 93% (Fragment IV). Sämtliche synthetischen Massnahmen der Fragmentherstellung und deren Kondensation am polymeren Träger werden fortlaufend mit einem Schreiber photometrisch über die spektroskopischen Eigenschaften der säurelabilen, temporär N-terminalen Ddz-Schutzgruppe überwacht (C. Birr in Y. Wolman: Peptides 1974, Halsted Press, New York (1975), Seite 117 ff).

Anstelle von Asn und Gln werden in den Positionen 5, 15 und 18 Asp (OBzl) bzw. Glu (OBzl) eingesetzt, um eine Nitrilbildung an den Amid-Seitenfunktionen durch das Dicyclohexylcarbodiimid-Kondensationsreagens zu vermeiden. In dem C-terminalen Fragment IV wird Asp (OH)$OBu^t$ mit seiner β-Carboxylfunktion über eine elektrophile 2-Oxoäthyl-esterbindung an die Aromaten des Polystyrolträgers gebunden. In dieser Weise können am Ende der Synthese alle zukünftigen Amidseitenfunktionen gleichzeitig durch eine Ammonolyse der bezeichneten Benzyl- und 2-Oxoäthylesterbindung bei der Ablösung der Fragmente IV bzw. V am polymeren Träger (siehe Fig. 4) eingeführt werden.

Alle übrigen Seitenfunktionen der Insulin A-Kette werden während der Synthese der Fragmente durch tert.-Butylschutzgruppen sicher geschützt. Durch Kondensation des Fragments I in 15fachem Überschuss mit Hilfe des Kondensationsreagens Dicyclohexylcarbodiimid in Dimethylformamid mit dem trägergebundenen Fragment II wird das Fragment III (1–14) am Träger in einer Ausbeute von 86% (bezogen auf das II-Gelpolymer) gebildet. Durch basische Spaltung der 2-Oxoäthylesterankerbindung des Fragments III an den Polystyrolträger (0,5 n-Triäthylamin in Methanol/Dioxan (1/1, Volumen/Volumen) + 0,5 Vol.-% in 1n wässrige Natriumhydroxidlösung) und anschliessende chromatographische Reinigung der abgelösten, voll geschützten Peptidsäure erhält man 1 g des reinen Fragments III (Ausbeute = 59% (bezogen auf das III-Gelpolymere)). Die unter den Abspaltbedingungen stabile Existenz des Benzylesters in der Position $A^5$ wurde über das Massenspektrum bewiesen. Das C-terminale Fragment IV wird mit mit Ammoniak gesättigtem Dioxan, dann mit einer flüssigen Ammoniak/Methanol-Mischung (4/1, Volumen/Volumen) unter Druck bei 20 °C vom Träger abgespalten. Nach der chromatographischen Reinigung (DEAE A 25 Sephadex®/Methanol-0,5n Essigsäure (4/1, Volumen/Volumen) und Sephadex LG 20®/Methanol) erhält man 1,45 g des reinen Fragments IV (Ausbeute = 86%, bezogen auf die trägergebundene Vorstufe). Bei Modellversuchen hat sich gezeigt, dass unter den genannten Bedingungen C-terminal das β-Amid des Asparaginsäure-α-tert.-butylesters entsteht und nicht das Succinimidderivat.

Die Kondensation von 0,5 mMol des Fragments III mit 1 mMol des Fragments IV zu dem voll geschützten Fragment V (1–21) mit Hilfe des Carbodiimid-Kondensationsreagens in Dimethylformamid (während 4 Tagen) ergibt nach chromatographischer Reinigung 1,3 g des Fragments V (was einer Ausbeute von 78%, bezogen auf das Fragment III, entspricht). Eine gleichartige Kondensation des Fragments III in 2,4fachem Überschuss mit dem Fragment IV am Träger unter Verwendung von Carbonyldiimidazol ergibt eine Ausbeute an dem Fragment V von 12%. Dabei können 50% des Überschusses des Fragments III zurückgewonnen werden.

In dem Fragment V wird der Benzylester in der Position $A^5$ mit Ammoniak in Methanol unter Druck in das Amid überführt.

Dann werden nach dem in der Fig. 4 dargestellten Schema die tert.-Butyldisulfide in den Positionen $A^6$ und $A^{11}$ reduktiv mit Tributylphosphan gespalten, und es wird selektiv der intrachenare Disulfidring $A^6$–$A^{11}$ durch Luftoxidation mit einer Ausbeute von 84% (bezogen auf das voll geschützte Fragment V) gebildet. Anschliessend werden sämtliche tert.-Butylester- und -äther-Schutzgruppen mit Trifluoressigsäure abgespalten. In diesem Zustand trägt die synthetische Insulin A-Kette nur noch die Schwefelschutzgruppen in den Positionen $A^7$ (Acm) und $A^{20}$ (Mbzl). Die p-Methoxybenzylschutzgruppe (Mbzl) wird selektiv mit Fluorwasserstoff in Pyridin in Gegenwart von Anisol abgespalten (mit einer Ausbeute von etwa 80%).

Die in dieser Weise erhaltene Insulin A-Kette mit einer einzigen Cysteinyl-Mercaptofunktion ($A^{20}$) wird sofort in 30%iger Essigsäure gelöst, mit der essigsauren Lösung einer äquimolaren Menge reduzierter, natürlicher B-Kette aus Rinderinsulin (zwei freie SH-Funktionen) vermischt und mit 0,1n Jodlösung oxidiert. Neben der angestrebten interchenaren Disulfidverbrückung ausschliesslich zwischen den Positionen $A^{20}$–$B^{19}$ wird hierbei als Sekundärschritt die letzte noch verbliebene Acetamidomethylschutzgruppe (Acm) in der Position $A^7$ jodolytisch entfernt, und es wird dann auch die zweite interchenare Disulfidbrücke ($A^7$–$B^7$) oxidativ gebildet.

Anschliessend wird sofort in 10%iger Essigsäure an Sephadex G 50® chromatographiert. Hierbei erhält man reines, elektrophoretisch und dünnschichtchromatographisch mit dem authentischen Vergleichsmaterial identisch wanderndes Rinderinsulin in einer Ausbeute von 25%. Das gefriergetrocknete Rohmaterial zeigt im biologischen Lipogenesetest an Fettzellen eine Aktivität von 52% und im kompetitiven Rezeptorbindungstest eine Aktivität von 65%.

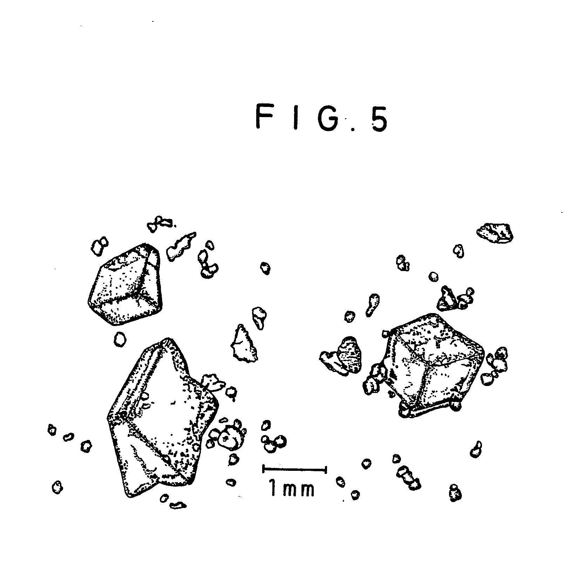

Das Material lässt sich als Zinkkomplex kristallisieren und zeigt die für Insulin charakteristische Kristallform (Fig. 5). Nach wiederholter Gelchromatographie an Biogel P 6 in 1% Essigsäure und der Ionenaustauschchromatographie an Carboxymethylcellulose zeigt das erfindungsgemäss gebildete halbsynthetische Rinderinsulin in den beiden oben genannten Testsystemen die volle biologische Aktivität.

Es ist somit ersichtlich, dass bei Anwendung der erfindungsgemässen selektiv abspaltbaren Schwefelschutzgruppen nur an der Insulin A-Kette sich die Ausbeute der Kombination bei Einsatz der Ketten im Verhältnis 1 : 1 gegenüber der vorbekannten statistischen Disulfidverbrückung bereits um mehr als 100% verbessern lässt. Diese Verfahrensweise lässt sich noch wesentlich verbessern, wenn auch die Positionen $B^7$ mit einer Acet-

amidomethylschutzgruppe und die Positionen $B^{19}$ mit einer p-Methoxybenzylschutzgruppe geschützt werden und dann in der erfindungsgemässen Verfahrensweise selektiv freigelegt werden können.

Das folgende Beispiel dient der weiteren Erläuterung der Erfindung.

Beispiel

Man führt die einzelnen Schritte der Peptidsynthese in einem Syntheseautomaten der Firma Schwarz Bio Research mit Hilfe eines Centrifugal Reactors durch. Die Umsatzkontrolle erfolgt durch Messung der UV-Absorption bei 280 nm und 230 nm und wird mit einem Zweikanalschreiber registriert.

a) Synthese des Fragments Insulin 1–3 (Fragment I) in Lösung

a)1 Synthese von ValOMe
$C_6H_{13}NO_2$ (131,173)

Man gibt 15,8 ml Thionylchlorid in 70 ml Methanol, das in einem Eis/NaCl-Gemisch auf −5 °C bis −10 °C abgekühlt ist. Dann trägt man 23,4 g Valin ein, wobei die Temperatur nicht über −5 °C ansteigen soll. Langsam wird auf 40 °C erwärmt, und es wird 2 Stunden bei dieser Temperatur gerührt. Man engt im Vakuum ein und nimmt in 5% $NaHCO_3$ auf. Dann schüttelt man mit Essigester aus, wäscht die Essigesterphase mit $H_2O$ neutral und trocknet mit $Na_2SO_4$. Nach dem Eindampfen im Vakuum bei 40 °C wird der weisse Rückstand im Exsikkator über $P_2O_5$ getrocknet.

Ausbeute: 19 g: 80%

Dünnschichtchromatogramm (n-Butanol/Eisessig/Wasser; 4/1/1): Rf = 0,46.

a)2 Ddz-Ile-ValOMe (gemischte Anhydridmethode)
$C_{24}H_{38}N_2O_7$ (466,577)

1. Bereitung der Aminkomponente
   Man löst 2,6 g (20 mMol) ValOMe in 50 ml $CH_2Cl_2$ und kühlt auf −15 °C.
2. Bereitung der Carboxylkomponente und Kupplung. Man löst 7,3 g (20 mMol) Ddz-Ile in 50 ml $CH_2Cl_2$ und versetzt mit 2,21 ml (20 mMol) N-Methylmorpholin. Anschliessend kühlt man auf −15 °C, aktiviert durch Zugabe von 2,24 ml (20 mMol) Chlorameisensäureisopropylester (Aktivierungszeit 8 Minuten) und gibt die Aminkomponente in einem Guss zu.
3. Aufarbeitung
   Das Reaktionsgemisch wird fünfmal mit eiskalter 0,1n $KHSO_4$-Lösung, einmal mit NaCl-gesättigtem Wasser und fünfmal mit $KHCO_3$-Lösung ausgeschüttelt. Das Lösungsmittel wird mit $Na_2SO_4$ getrocknet und im Vakuum abgezogen. Der verbliebene Rückstand wird über $P_2O_5$ getrocknet.

Ausbeute: 7,4 g: 80%

Dünnschichtchromatogramm (Benzol/Eisessig; 7/1) Rf = 0,39

a)3 Ddz-Gly-Ile-ValOMe
$C_{26}H_{41}N_3O_8$ (523,630)

1. Bereitung der Aminkomponente
   Man löst 7,4 g (16mMol) Ddz-Ile-ValOMe in 100 ml 5%iger Trifluoressigsäure in $CH_2Cl_2$ (Abspaltung der Schutzgruppe in 15 Minuten). Dann wird die Reaktionslösung mit N-Methylmorpholin neutralisiert (mit pH-Papier auf pH 7 bis 7,5) und auf −15 °C gekühlt.
2. Bereitung der Carboxylkomponente und Kupplung. Man löst 5,6 g (20 mMol) Ddz-Gly in 50 ml $CH_2Cl_2$ und versetzt mit 2,21 ml (20 mMol) N-Methylmorpholin. Anschliessend kühlt man auf −15 °C, aktiviert durch Zugabe von 2,24 ml (20 mMol) Chlorameisensäureisopropylester (Aktivierungszeit 8 Minuten) und gibt die Aminkomponente in einem Guss zu.
3. Aufarbeitung
   Das Reaktionsgemisch wird fünfmal mit eiskalter 0,1n $KHSO_4$-Lösung, einmal mit NaCl-gesättigtem Wasser und fünfmal mit $KHCO_3$-Lösung ausgeschüttelt. Das Lösungsmittel wird mit $Na_2SO_4$ getrocknet und im Vakuum abgezogen. Der verbliebene Rückstand wird über $P_2O_5$ getrocknet.

Ausbeute: 7,1 g: 88%
Schmelzpunkt: 147 °C

Aminosäureanalyse: HCl/Propionsäure
15 Minuten 160°C

| | Gly | Val | Ile |
|---|---|---|---|
| berechnet: | 1 | 1 | 1 |
| gefunden: | 1,10 | 1,00 | 1,01 |

Massenspektrum:
m/e = 523

a)4 Ddz-Gly-Ile-Val
$C_{25}H_{39}N_3O_8$ (509,604)

Man löst 7,1 g (14 mMol) Ddz-Gly-Ile-ValOMe in 20 ml Dioxan und versetzt mit 2 Äqu. 1n NaOH. Man rührt bei Zimmertemperatur, neutralisiert mit 1n Essigsäure, engt im Vakuum ein und reinigt durch Gelchromatographie (Sephadex LG-20®/Methanol).

Man verfolgt die Verseifung dünnschichtchromatographisch: vollständige Verseifung.

Dünnschichtchromatogramm (Chloroform/Methanol/Pyridin; 95/5/3): Rf = 0,10

Drehwert: $[\alpha]^{25}_{D}$ = +1,5° (C = 1,4) Methanol

b) Automatische Festphasensynthese von Insulin A-Kettenfragmenten 15–21 und 4–14 (IV bzw. II)

| | |
|---|---|
| 1. 1 × 2 Min. | Ddz+)-Abspaltung mit 5% Trifluoressigsäure in $CH_2Cl_2$ |
| 2. 1 × 15 Min. | |
| 3. 6 × je 2 Min. | Waschen mit $CH_2Cl_2$ |
| 4. 1 × 2 Min. | Wiederholung der Ddz-Abspaltung |
| 5. 1 × 15 Min. | mit 5% Trifluoressigsäure |
| 5. 6 × je 2 Min. | Waschen mit $CH_2Cl_2$ |

1–6. Das Filtrat des Abspaltungsproduktes und die Waschflüssigkeit werden gesammelt zur photometrischen Bestimmung des Ddz-Spaltprodukts;

Die Umsatzkontrolle.

7. 4 × je 2Min. Reaktion mit Triäthylamin [$CH_2Cl_2$/Dimethylformamid (1/1)] 1 : 9
8. 12 × je 2 Min. Waschen mit $CH_2Cl_2$
9. 1 × Zugabe von 20 ml Ddz-Aminosäurelösung (berechnet auf vorhergehende Ddz-Werte) in $CH_2Cl_2$
10. 1 × Zugabe von 20 ml Dicyclohexylcarbodiimid-Lösung (gleiche Äquivalente wie die Ddz-Aminosäure) in Dimethylformamid
11. 1 × Zugabe von 20 ml Dimethylformamid/$CH_2Cl_2$ (1/1)
12. 1 × 60 Min. Reaktionszeit
13. 3 × 2 Min Auswaschen mit $CH_2Cl_2$
14. 5 × 2 Min. Auswaschen mit $CH_2Cl_2$/Methanol
15. 4 × 2 Min. Auswaschen mit $CH_2Cl_2$
16. Wiederholung der Schritte 9–15 zweimal

+) Ddz = α,α-Dimethyl-3,5-dimethoxy-benzyloxycarbonylgruppe

17. 1 × Zugabe von 40 ml 0,1 M 3-Nitrophthalsäureanhydrid in Pyridin
18. 1 × Zugabe von 20 ml Dimethylformamid/$CH_2Cl_2$ (1/1)
19. 1 × 10 Min. Reaktionszeit
20 8 × 2 Min. $CH_2Cl_2$ Auswaschen
21. 5 × 2 Min. Auswaschen mit $CH_2Cl_2$/Methanol (4/1)
22. 4 × 2 Min. $CH_2Cl_2$
23. 5 × 2 Min. Auswaschen mit $CH_2Cl_2$/Methanol (4/1)
24. 4 × 2 Min. Auswaschen mit $CH_2Cl_2$.

Das oben beschriebene Syntheseprogramm wird für die Synthese von Insulin A 15–21 (Fragment IV) und A 4–14 (Fragment II) verwendet. Die jeweilige Carboxylkomponente (Ddz-Aminosäure) wird in jedem Zyklus in vierfachem Überschuss, bezogen auf den vorhergehenden Ddz-Wert, eingesetzt. Als Lösungsmittel und Waschflüssigkeit für Schritte 1 bis 9 dient über eine $Al_2O_3$-Säule gereinigtes Methylenchlorid. Die Synthese wird mit 3 g Ddz-AspOBu$^t$-Polystyrol (mit 0,5% Divinylbenzol vernetzt) und 3 g Ddz-Tyr(Bu$^t$)-Polystyrol (mit 0,5% Divinylbenzol vernetzt) im Zentrifugalreaktor gestartet.

Beladungen:
0,526 mMol Ddz-AspOBu$^t$/g Polymer
0,664 mMol Ddz-AspOBu$^t$/g Polymer
0,900 mMol Ddz-Tyr(Bu$^t$)/g Polymer
0,857 mMol Ddz-Tyr(Bu$^t$)/g Polymer

Wenn im Programm kein Volumen angegeben ist, wird an dieser Stelle von dem Syntheseautomaten ein Volumen von 60 ml abgemessen.

Die Umsätze, bezogen auf die Ausgangsbeladung, werden auf der Basis der photometrischen Vermessung des Ddz-Spaltprodukts wie folgt gefunden:

Umsatzergebnisse Insulin 15–21

| | 1. Synthese Beladung mMol/g Träger | Umsatz % | 2. Synthese Beladung mMol/g Träger | Umsatz % |
|---|---|---|---|---|
| AspOBu$^t$ | 0,526 | – | 0,664 | – |
| Cys (Mbzl) | 0,529 | 100 | 0,714 | 100 |
| Tyr (Bu$^t$) | 0,412 | 78 | 0,660 | 100 |
| Asp (OBzl) | 0,358 | 92 | 0,670 | 100 |
| Glu (OBu$^t$) | 0,308 | 86 | 0,593 | 90 |
| Leu | 0,250 | 81 | 0,563 | 95 |
| Glu (OBzl) | 0,240 | 96 | 0,600 | 100 |

Siehe Fig. 2. Die Durchschnittsausbeute beider Synthesen beträgt 93%.

Umsatzergebnisse Insulin 4–14

| | 1. Synthese Beladung mMol/g Träger | Umsatz % | 2. Synthese Beladung mMol/g Träger | Umsatz % |
|---|---|---|---|---|
| Tyr (Bu$^t$) | 0,900 | – | 0,857 | – |
| Leu | 0,523 | 58 | 0,600 | 70 |
| Ser (Bu$^t$) | 0,502 | 96 | 0,591 | 97 |
| Cys (SBu$^t$) | 0,491 | 98 | 0,571 | 98 |
| Val | 0,470 | 96 | 0,442 | 77 |
| Ser (Bu$^t$) | 0,463 | 99 | 0,400 | 90 |
| Ala | 0,470 | 100 | 0,414 | 100 |

| | 1. Synthese Beladung mMol/g Träger | Umsatz % | 2. Synthese Beladung mMol/g Träger | Umsatz % |
|---|---|---|---|---|
| Cys (Acm) | 0,466 | 99 | 0,407 | 98 |
| Cys ($SBu^t$) | 0,500 | 100 | 0,392 | 96 |
| Glu (OBzl) | 0,433 | 86 | 0,352 | 89 |
| Glu ($OBu^t$) | 0,300 | 69 | 0,300 | 86 |

Siehe Fig. 3. Die Durchschnittsausbeute beider Synthesen beträgt 90%.

Die zweite Synthese wird nicht nach dem 10. Zyklus abgebrochen, sondern es erfolgt die Umsetzung mit Insulin A 1–3. Das Tripeptid wird jeweils in 5fachem Überschuss bei drei Umsetzungen eingesetzt. Für diese Reaktion wird das Syntheseprogramm wie folgt geändert:

| | | |
|---|---|---|
| 9. 1 × Zugabe von | 20 ml | Ddz-Tripeptidlösung ($CH_2Cl_2$) |
| 10. 1 × Zugabe von | 20 ml | 1-Hydroxybenzotriazol-Lösung (in Dimethylformamid) |
| 11. 1 × Zugabe von | 10 ml | Dicyclohexylcarbodiimid-Lösung in Dimethylformamid/$CH_2/Cl_2$ (1/1) |
| 12 1 × Zugabe von | 20 ml | $CH_2Cl_2$ Dimethylformamid (1/1) |
| 13. 1 × 4 Stunden | | Reaktionszeit |
| 14. 3 × 2 Min. | | Auswaschen mit $CH_2Cl_2$ |
| 15. 5 × 2 Min. | | Auswaschen mit $CH_2Cl_2$/Methanol (4/1) |
| 16. 4 × 2 Min. | | Auswaschen mit $CH_2Cl_2$ |

17. Wiederholung der Schritte 9 bis 16 zweimal.

Bei der Fragmentkondensation werden in jedem Zyklus 15,75 mMol Tripeptid, 1 Äquivalent Dicyclohexylcarbodiimid und 1,5 Äquivalente 1-Hydroxybenzotriazol eingesetzt.

Umsatzergebnis Insulin 1–14 (Fragment III)

| | Beladung mMol/g Träger | Umsatz % |
|---|---|---|
| Insulin A 4–14 | 0,300 | – |
| Insulin A 1–14 | 0,258 | 86 |

c) Synthese der Insulin A-Kette am polymeren Träger

c)1 Automatisierte Abspaltung der Insulin-Fragmente II (4–14), III (1–14) und IV (15–21) vom polymeren Träger

Die Abspaltung erfolgt mit modifiziertem Beyerman-Reagens (1n Triäthylamin/Methanol + 1% 1n wässrige NaOH)/Dioxan (1 : 1) mit folgendem Syntheseprogramm:

| | |
|---|---|
| 1. 1 × 2 Min. | Peptidabspaltung |
| 2. 3 × 4 Min. | Peptidabspaltung |
| 3. 20 × 30 Min. | Peptidabspaltung |

Die ganze Abspaltungsreaktion wird kontinuierlich durch die photometrische Überwachung aufgezeichnet. An der Aufzeichnung lässt sich das Ende der Abspaltungsreaktion ablesen. Meist ist die Abspaltung nach etwa 3 bis 6 Stunden beendet. Das Filtrat wird in einem Kolben aufgefangen, in dem $CO_2$-Trockeneis zur Neutralisation der basischen Peptidlösung vorgelegt worden ist. Da bei der Synthese 3-Nitrophthalsäure-anhydrid als Blocker verwendet wird, können die Rumpfsequenzen durch eine erste Fraktionierung über DEAE-Ionenaustauscher A-25® mit einer Mischung aus Methanol/0,5 n Essigsäure (4/1) abgetrennt werden. Danach wird eine Reinigung durch Säulenchromatographie über Sephadex LH-20® (2,5 × 200) mit Methanol als Laufmittel durchgeführt.

c)2 Ddz-Glu($OBu^t$)-Glu(OBzl)-Cys($SBu^t$)-Cys-(ACm)-Ala-Ser($Bu^t$)-Val-Cys($SBu^t$)-Ser($Bu^t$)-Leu-Tyr($Bu^t$) [1] $C_{94}H_{148}N_{11}O_{24}$ (1816,285)

Nach der oben beschriebenen Abspaltung und Reinigung wird das Gemisch aus Methylester und freier Säure des Fragments II (4–14) einer Verseifung unterworfen.

Ca. 1,1 g des Fragments II (4–14) werden in 3 ml Dioxan gelöst und mit zwei Äquivalenten 1n NaOH versetzt. Der pH der Lösung wird mit einem pH-Stat auf 11,5 gehalten. Nach 4 Stunden bei Raumtemperatur ist die Reaktion abgeschlossen. Dann wird mit 1n Essigsäure neutralisiert und im Vakuum eingeengt. Anschliessend erfolgt die Entsalzung durch Gelchromatographie über Sephadex LH-20®/Methanol. Zum Schluss wird über $P_2O_5$ getrocknet. Ausbeute 73% (1,1 g), bezogen auf den letzten Ddz-Wert der N-terminalen Aminosäure des trägergebundenen Heptapeptids.

Dünnschichtchromatogramm (n-Butanol/Eisessig/Wasser; 4/1/1): Rf = 0,83

Massenspektroskopischer Nachweis der Schutzgruppen:

| Spaltstück von | Ddz | Acm | OBzl |
|---|---|---|---|
| m/e | 178 | 72 | 107 |

Aminosäureanalyse: (HCl/Propionsäure, 15 Minuten, 160 °C)

| | Cys CysSO$_3$H | Ser | Glu | Ala | Val | Leu | Tyr |
|---|---|---|---|---|---|---|---|
| berechnet: | 3 | 2 | 2 | 1 | 1 | 1 | 1 |
| gefunden: | 2,38 | 1,82 | 1,64 | 1,10 | 1,13 | 1,00 a) | 0,95 |

a) Bezugsaminosäure
Drehwert: $[\alpha]^{25}_{D} = -38{,}2$ (C = 0,5) MeOH

c)3 Ddz-Gly-Ile-Val-Glu(OBu$^t$)-Glu(OBzl)-Cys(S-Bu$^t$)-Cys(Acm)-Ala-Ser(Bu$^t$)-Val-Cys(SBu$^t$)-Ser(Bu$^t$)-Leu-Tyr(Bu$^t$). [2] $C_{107}H_{171}N_{14}O_{27}$ (2085,634)

Nach der obigen allgemein beschriebenen Abspaltung und den Reinigungsoperationen erfolgt noch eine Trennung des Fragments III (1–14) [2] über Sephadex LH-60®/Methanol. Das auch hierdurch nicht aufgetrennte Gemisch zwischen Methylester und freier Säure wird wiederum analog Fragment II [1] verseift.

Ausbeute: 59% (1,1 g), bezogen auf den letzten Ddz-Wert der N-terminalen Aminosäure des trägergebundenen Heptapeptids.

Dünnschichtchromatogramm (Chloroform/Methanol/Eisessig; 85/15/5): Rf = 0,63 (einheitlich).

Massenspektroskopischer Nachweis der Schutzgruppen:

| Spaltstück von | Ddz | Acm | OBzl | Bu$^t$ |
|---|---|---|---|---|
| m/e | 178 | 72 | 107 | 57 |

Aminosäureanalyse: 6n HCl, 28 Stunden, 110 °C.

| | Cys CysSO$_3$H | Ser | Glu | Gly | Ala | Val | Ile | Leu | Tyr |
|---|---|---|---|---|---|---|---|---|---|
| berechnet: | 3 | 2 | 2 | 1 | 1 | 2 | 1 | 1 | 1 |
| gefunden: | 2,86 | 2,08 | 1,78 | 1,05 | 1,18 | 2,04 | 1,03 | 1,00 a) | 0,86 |

a) Bezugsaminosäure
Drehwert: $[\alpha]^{25}_{D} = -32{,}3$ (C = 0,6 Methanol)

Dieses Fragment wird später für die Kondensation in Lösung eingesetzt.

d)4 Ddz-Glu(OBzl)-Leu-Glu(OBu$^t$)-Asp(OBzl)-Tyr(Bu$^t$)-Cys(MBzl)-AspOBu$^t$ [3] $C_{81}H_{109}N_7O_{22}$ (1532,803)

Nach der Abspaltung und Reinigung des Fragments IV (15–21) [3] wird die Verseifung analog Fragment II [1] durchgeführt.

Ausbeute 51% (0,5 g), bezogen auf den letzten Ddz-Wert der N-terminalen Aminosäure des trägergebundenen Heptapeptids.

Dünnschichtchromatogramm (n-Butanol/Eisessig/Wasser; 4/1/1): Rf = 0,80

Massenspektrometrischer Nachweis der Schutzgruppen:

| Spaltstück von | Ddz | OBzl | MBzl | Bu$^t$ |
|---|---|---|---|---|
| m/e | 178 | 107 | 121 | 57 |

Aminosäureanalyse: HCl/Propionsäure, 15 Minuten, 160 °C

| | Asp | Glu | Leu | Tyr | Cys |
|---|---|---|---|---|---|
| berechnet: | 2 | 2 | 1 | 1 | 1 |
| gefunden: | 1,67 | 1,70 | 1,00 a) | 0,87 | 0,83 |

a) Bezugsaminosäure
Drehwert: $[\alpha]^{25}_{D} = -41{,}2°$ (C = 2 Methanol)

c)5 Wiederankupplung des Fragments IV (Insulin 15 bis 21) am polymeren Träger

$-O-CH_2-CO-$

Ddz-Glu(OBzl)-Leu-Glu(OBu$^t$)-Asp(OBzl)-Tyr(Bu$^t$)–Cys(MBzl)–AspOBu$^t$ [4]

Man löst 400 mg (0,26 mMol) Insulin A 15–21 [3] in 5 ml Methanol. Dann gibt man 38,4 mg (0,23 mMol) CsOH-Lösung (10% Unterschuss) zu. Das Lösungsmittel wird im Vakuum abgezogen und der Rückstand über $P_2O_5$ getrocknet.

Das getrocknete Caesiumsalz wird zur Harzveresterung in absolutem Dimethylformamid gelöst in im 5fachen Überschuss zum Harz (1,89 mMol Br/g Harz) gegeben (634 mg). Der Ansatz wird 3 Tage bei 40 °C gerührt. Das Harz wird über eine Glasfritte abfiltriert und gründlich mit folgenden Lösungsmitteln gewaschen: Dimethylformamid, Chloroform, Dioxan/Methanol (3/1), Methanol, Dioxan und Methanol. Danach wird es im Exsikkator über $P_2O_5$ getrocknet. Die Beladung des Harzes wird mittels Elementaranalyse auf Stickstoff, durch photometrische Analyse der Ddz-Schutzgruppenabspaltung und quantitative Aminosäureanalyse bestimmt. Eine quantitative Aminosäureanalyse ergab eine Beladung von 0,11 mMol/g Harz.

c)6 Wiedergewinnung des Fragments IV (15–21)

Die verschiedenen Waschlösungen werden vereinigt und im Vakuum auf einem Rotationsverdampfer eingedampft. Der Rückstand wird in Essigester aufgenommen und durch Ausschütteln mit 0,5n $KHSO_4$-Lösung in die freie Säure überführt. Die organische Phase wird dann mit $H_2O$ neutral gewaschen und über $Na_2SO_4$ getrocknet. Anschliessend wird im Vakuum in einem Rotationsverdampfer eingedampft und über $P_2O_5$ getrocknet. Man erhält 200 mg der dünnschichtchromatographisch reinen Fraktion IV (Insulin 15–21) zurück.

c)7 Fragment-Kondensation zu dem Fragment III (Insulin 1–14) in Lösung

Ddz-Gly-Ile-Val-Glu(OBu$^t$)-Blu(OBzl)-Cys (SBu$^t$)-
Cys(Acm)-Ala-Ser(Bu$^t$)-Val-Cys(SBu$^t$)-Ser(Bu$^t$)-Leu-Tyr(Bu$^t$) [5] $C_{107}H_{171}N_{14}O_{27}$ (2085,634)

Man löst 763 mg (1,5 mMol) der Fraktion I (Insulin A 1–3), 270 mg (2mMol) 1-Hydroxybenzotriazol und 0,22 ml (2mMol) N-Methylmorpholin in 20 ml absolutem Dimethylformamid. Die Lösung wird auf 0 °C gekühlt und mit 206 mg (1,0 mMol) Dicyclohexylcarbodiimid versetzt. Man rührt 1 Stunde bei 0 °C und gibt dann 600 mg (0,35 mMol) des Fragments II (Insulin A 4–14 [1]) (auf pH 7,5 eingestellt mit N-Methylmorpholin) zu. Man rührt 12 Stunden über Nacht, engt dann im Vakuum ein und reinigt durch Gelchromatographie (Sephadex LH-20®/Methanol).

Die Ausbeute beträgt 413 mg des Fragments III (Insulin A 1–14), was einem Umsatz von 52% entspricht.

Aminosäureanalyse: HCl/Propionsäure, 20 Minuten, 160 °C

| | Cys CysSO$_3$H | Ser | Glu | Gly | Ala | Val | Ile | Leu | Tyr |
|---|---|---|---|---|---|---|---|---|---|
| berechnet: | 3 | 2 | 2 | 1 | 1 | 2 | 1 | 1 | 1 |
| gefunden: | 1,66 | 1,64 | 1,99 | 1,02 | 1,00a) | 2,21 | 0,93 | 1,04 | 0,65 |

a) Bezugsaminosäure

Drehwert: $[a]^{25}_D = -32,1$ (C = 0,1 Methanol)

c)8 Fragment-Kondensation zu dem Fragment V (Insulin 1–21) am polymeren Träger

Ddz-Gly-Ile-Val-Glu(OBu$^t$)-Blu(OBzl)-Cys (SBu$^t$)-
Cys(Acm)-Ala-Ser(Bu$^t$)-Val-Cys(SBu$^t$)-Ser(Bu$^t$)-
Leu-Tyr(Bu$^t$)-Glu(OBzl)-Leu-Glu(OBu$^t$)-Asp(O-Bu$^t$)-

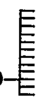

–O–$CH_2$–CO–

Tyr(Bu$^t$)–Cys(MBzl)–AspOBu$^t$ [6]

Man gibt 700 mg Insulin 15–21-Phenacetylharz [4] (Beladung 0,074 mMol) in eine Schüttelfritte und versetzt mit 5%iger Trifluoressigsäure in $CH_2Cl_2$. Man schüttelt eine halbe Stunde und wäscht dann gründlich mit $CH_2Cl_2$. Der Vorgang wird zweimal wiederholt. Die Schüttelfritte wird danach über $P_2O_5$ getrocknet. Das Peptid wird in der Schüttelfritte mit Triäthylamin [$CH_2Cl_2$/Dimethylformamid (1 : 2)] (1 : 3) deprotoniert und gründlich mit Dimethylformamid/$CH_2Cl_2$ und Dimethylformamid gewaschen.

Man löst 400 mg (0,18 mMol) des Fragments III (Insulin 1–14 [5] und 28 mg (0,17 mMol) Carbonyldiimidazol bei 0 °C in 5 ml absolutem Dimethylformamid und setzt es dem obigen trägergebundenen Fragment IV (15–21) zu. Man rührt 4 Tage, filtriert die Reaktionslösung ab und trocknet das Harz über $P_2O_5$. Das Fragment III (Insulin 1–14) kann zurückgewonnen werden, wie weiter unten beschrieben wird. Eine quantitative Aminosäureanalyse, bezogen auf Alanin in dem Fragment III (Insulin 1–14), ergibt eine Beladung von 0,0086 mMol, das sind 30 mg Insulin 1–21. Dies entspricht einem Umsatz von 11,6%. Die Umsetzung wird wiederholt, wobei eine Erhöhung des Umsatzes nicht erreicht werden konnte.

Wiedergewinnung des Fragments III (Insulin 1–14)

Die verschiedenen Waschlösungen werden vereinigt und im Vakuum in einem Rotationsverdampfer eingedampft. Durch Wasserzugabe wird überschüssiges Imidazolid in die freie Säure überführt. Es wird wiederum eingedampft (Rotationsverdampfer) und getrocknet. Der Rückstand wird durch Gelchromatographie über Sephadex LH-20® gereinigt. Man erhält 210 mg des Fragments III (Insulin 1–14) zurück.

c)9 Abspaltung der Insulin A-Kette vom polymeren Träger

Ddz-Gly-Ile-Val-Glu(OBu$^t$)-Gln-Cys(SBu$^t$)-Cys(Acm)-Ala-Ser(Bu$^t$)-Val-Cys(SBu$^t$)-Ser(Bu$^t$)-Leu-Tyr(Bu$^t$)-Gln-Leu-Glu(OBu$^t$)-Asn-Tyr(Bu$^t$)-Cys(MBzl)-AsnOBu$^t$. [7]

Der polymere Träger [6] wird in einer Stahlbombe mit 10 ml Methanol aufgeschlämmt Unter Feuchtigkeitsausschluss wird die Bombe gekühlt und mit 80 ml flüssigem Ammoniak versetzt. Auf diese Weise sollen die β-Carboxylfunktion aus ihrer Bindung zum Phenacetylträger als auch die β-Benzylestergruppierungen der Asparaginsäure in Position 18 und die γ-Benzylestergruppierung der Glutaminsäure in Position 5 und 15 gespalten und in einem Schritt in die entsprechenden Amide überführt werden. Die Bombe wird verschlossen und 3 Tage bei Zimmertemperatur geschüttelt.

Nach der Abspaltungsreaktion wird das Harz abfiltriert und gründlich mit Methanol gewaschen. Die Aminosäureanalyse einer Harzprobe in 6n HCl bei 160 °C, 20 Minuten, zeigt, dass die Abspaltung vollständig verlaufen ist. Die Abspaltlösung wird im Vakuum bei 40 °C eingedampft und in Methanol aufgenommen. Nach der dünnschichtchromatographischen Kontrolle enthält die Abspaltungslösung neben dem Zielprodukt Insulin A-Kette den nichtumgesetzten Anteil des Fragments IV (15–21). Es verbleibt ausserdem ein unlöslicher Rückstand, der auch in Essigester nicht aufzulösen ist. Von diesem festen Rückstand wird eine Aminosäureanalyse durchgeführt. Die Analyse ergibt, dass der unlösliche Anteil aus Insulin A-Kette besteht. Da aber ein Mercaptangeruch festgestellt werden kann, ist anzunehmen, dass die S-tert.-Butylschutzgruppen zum Teil durch die basischen Bedingungen abgespalten worden sind und der feste Rückstand aus oligomerer Insulin A-Kette besteht (20 mg). Der feste Rückstand und die Methanollösung werden vereinigt und zur weiteren Reaktion eingesetzt. Das Gemisch wird über $P_2O_5$ getrocknet.

Rohausbeute (Methanol-Phase) ca. 80 mg

Unlöslicher Rückstand ca. 20 mg

Dünnschichtchromatrogramm (n-Butanol/Eisessig/Wasser; 4/1/1): Rf = 0,9 (Fragment 15–21), 0,2 (Insulin A-Kette)

c)10 Bildung der intrachenaren Disulfidbrücke $A^6$–$A^{11}$

Ddz-Gly-Ile-Val-Glu(OBu$^t$)-Gln-Cys-Cys(Acm)-Ala-Ser(Bu$^t$)-Val-Cys-Ser(Bu$^t$)-Leu-Tyr(Bu$^t$)-Gln-Leu-Glu)OBu$^t$)-Asn-Tyr(Bu$^t$)-Cys(Mbzl)-AsnOBu$^t$. [8]

Das Peptidgemisch [7] aus der letzten Abspaltung (c)9) wird für die Reduktion von Cys 6 und Cys 20 eingesetzt. Die Reduktion wird mit Tributylphosphan durchgeführt. Anschliessend erfolgt die Schliessung der Disulfidbindungen durch Luftoxidation.

30 mg der Fraktion V (Insulin 1–21) ca. 100 mg Gemisch [7] werden in 2,0 ml Propanol/Wasser gelöst und mit Stickstoff durchströmt. Dann erfolgt die Zugabe von 4 µl Tributylphosphan. Die Reaktionslösung lässt man 48 Stunden unter Stickstoff bei Zimmertemperatur reagieren.

Danach wird die gesamte Reaktionslösung in 1 l Wasser, das mit Ammoniak auf pH 8 eingestellt worden ist, gegeben. Durch die Lösung leitet man Luft während 48 Stunden. Anschliessend wird im Vakuum eingeengt und durch Gelchromatographie über Sephadex LH-20® getrennt. Man erhält 20 mg reine, voll geschützte Insulin A-Kette [8].

Die Ausbeute der Schliessung des inneren Rings beträgt 66%.

Aminosäureanalyse: HCl/Propionsäure, 35 Minuten, 160 °C.

| | Cys $CysSO_3H$ | Asp | Ser | Glu | Ala | Val | Ile | Leu | Tyr | Gly |
|---|---|---|---|---|---|---|---|---|---|---|
| berechnet: | 4 | 2 | 2 | 4 | 1 | 2 | 1 | 2 | 2 | 1 |
| gefunden: | 3,05 | 1,86 | 2,12 | 4,10 | 1,03 | 2,00a) | 0,87 | 2,24 | 1,87 | 0,97 |

a) Bezugsaminosäure

Drehwert: $[\alpha]^{25}_D = -42°$ (C = 0,1) Methanol

d) Synthese von Insulin A-Kette in Lösung

d)1 Abspaltung des Fragments IV (Insulin A 15–21) vom polymeren Träger

Ddz-Gln-Leu-Glu(OBu$^t$)-Asn-Tyr(Bu$^t$)-Cys(MBzl)-AsnOBu$^t$. [9] $C_{67}H_{100}N_{10}O_{19}$ (1349,560)

Die Abspaltung, bei der sowohl die β-Carboxylfunktion aus ihrer Bindung zum Phenacetylträger als auch die β-Benzylestergruppierung der Asparaginsäure in Position 18 in das Amid überführt werden, erfolgt mit Dioxan/$NH_3$. Die ganze Abspaltung wird kontinuierlich über ein UV-Photometer überwacht und vom Schreiber aufgezeichnet. Das Ende wird nach zwei Tagen angezeigt. Eine quantitative Aminosäureanalyse ergibt, dass nur 50% Peptid vom Träger abgespalten worden sind.

Der polymere Träger wird dann in einer Stahlbombe mit 10 ml Methanol aufgeschlämmt. Unter Feuchtigkeitsausschluss wird die Bombe auf −70 °C heruntergekühlt und mit 80 ml flüssigem Ammoniak versetzt. Die Bombe wird verschlossen und 3 Tage bei Zimmertemperatur geschüttelt. Nach der Abspaltungsreaktion wird das Harz abfiltriert und gründlich mit Methanol gewaschen. Eine Aminosäureanalyse in 6n HCl bei 160 °C/20 Minuten zeigt, dass die Abspaltung vollständig verlaufen ist. Die verschiedenen Abspaltungsreaktionen werden wie folgt aufgearbeitet.

Anschliessend wird eine Fraktionierung an DEAE-A 25 Ionenaustauscher mit einer Mischung aus Methanol/0,5n Essigsäure (4/1) durchgeführt. Danach erfolgt die Reinigung durch Säulenchromatographie über Sephadex LH-20®-Säule (2,5 × 200 cm) mit Methanol als Laufmittel. Das Produkt der Abspaltung mit flüssigem Ammoniak enthält zwei Flecken, die in der Elektrophorese und auf der DC-Platte verschieden laufen. Es kann durch Dansylierung gezeigt werden, dass von einem Teil des Peptids die Ddz-Schutzgruppe abgespalten worden ist.

Ausbeute bei der Abspaltung mit Dioxan/Ammoniak 700 mg (42%), bezogen auf den letzten Ddz-Wert der N-terminalen Aminosäure des trägergebundenen Heptapeptids.

Dünnschichtchromatogramm (Chloroform/Methanol/Eisessig; 85/15/5): Rf = 0,7

Dünnschichtchromatogramm (n-Butanol/Eisessig/Wasser; 4/1/1): Rf = 0,9

Die Ausbeute bei der Abspaltung mit flüssigem Ammoniak unter Druck beträgt 0,75 g (44%), bezogen auf den letzten Ddz-Wert der N-terminalen Aminosäure des trägergebundenen Heptapeptids.

Dünnschichtchromatogramm (Chloroform/Methanol/Eisessig; 85/15/5): Rf = 0,7 und 0,5

Dünnschichtchromatogramm (n-Butanol/Eisessig/Wasser; 4/1/1): Rf = 0,9 und 0,8

Die Gesamtausbeute beträgt 86% (1,45 g), bezogen auf den letzten Ddz-Wert der N-terminalen Aminosäure des trägergebundenen Heptapeptids.

Aminosäureanalyse: HCl/Propionsäure, 15 Minuten, 160 °C

| | Aps | Glu | Leu | Tyr | Cys |
|---|---|---|---|---|---|
| berechnet: | 2 | 2 | 1 | 1 | 1 |
| gefunden: | 1,91 | 1,84 | 1,00a) | 0,76 | 0,58 |

a) Bezugsaminosäure

Das Pherogramm bei pH 1,9 1 h 1000 V ergibt am Start und ca. 2 cm von der Startlinie entfernt auf der Anodenseite jeweils eine breite Bande, die beide Ninhydrin-positiv reagieren.

d)2 Fragment-Kondensation zu Insulin A-Kette in Lösung

Ddz-Gly-Ile-Val-Glu(OBu$^t$)-Glu(OBzl)-Cys(SBu$^t$)-Cys(Acm)-Ala-Ser(Bu$^t$)-Val-Cys(SBu$^t$)-Ser(Bu$^t$)-Leu-Tyr(Bu$^t$)-Gln-Leu-Glu(OBu$^t$)-Asn-Tyr(Bu$^t$)-Cys(MBzl)-AsnOBu$^t$. [10]

Man löst 1,1 g (0,5 mMol) des in Abschnitt c)3 beschriebenen Insulins A 1–14 [2] in 20 ml absolutem Dimethylformamid. Die Reaktionslösung wird auf −10 °C heruntergekühlt und mit 1 Äquivalent (103 mg) Dicyclohexylcarbodiimid versetzt. Das Gemisch wird 10 Minuten bei −10 °C gerührt. Danach werden 2 Äquivalente 1-Hydroxybenzotriazol (135 mg) und 2 Äquivalente N-Methylmorpholin (0,11 ml) zugegeben und die Temperatur auf 0 °C ansteigen gelassen (ca. 10 Minuten). Bei 0 °C werden dann 2 Äquivalente (1,16 g) der Aminkomponente [9] und 2 Äquivalente N-Methylmorpholin (0,11 ml) zugegeben, das Gemisch auf Raumtemperatur erwärmt und 4 Tage gerührt.

Danach wird die Reaktionslösung im Vakuum eingeengt und durch Gelchromatographie über Sephadex LH-20® mit Methanol und über Kieselgel K-60 (Fertigsäule) mit einem Chloroform/Methanol-Gradienten getrennt.

Die Ausbeute beträgt 1,3 g vollgeschützte A-Kette, was einem Umsatz von 78% entspricht, bezogen auf das Fragment III (1–14).

Dünnschichtchromatogramm (n-Butanol/Eisessig/Wasser; 4/1/1): Rf = 0,2

Die Aminosäureanalyse (6n HCl, 64 Stunden, 110 °C) ergab:

| | Cys CysSO$_3$H | Asp | Ser | Glu | Gly | Ala | Val | Ile | Leu | Tyr |
|---|---|---|---|---|---|---|---|---|---|---|
| berechnet: | 4 | 2 | 2 | 4 | 1 | 1 | 2 | 1 | 2 | 2 |
| gefunden: | 4,12 | 2,21 | 1,86 | 3,94 | 1,01 | 1,02 | 1,00a) | 0,96 | 2,24 | 1,86 |

a) Bezugsaminosäure

Drehwert: $[\alpha]^{25}_{D}$ = −37,3 (C = 0,05) MeOH

d)3 Überführung von Glu(OBzl) in Gln in Position 5

Ddz-Gly-Ile-Val-Glu(OBu$^t$)-Gln-Cys(SBu$^t$)-Cys(Acm)-Ala-Ser(Bu$^t$)-Val-Cys(SBu$^t$)-Ser(Bu$^t$)-Leu-Tyr(Bu$^t$)-Gln-Leu-Glu(OBu$^t$)-Asn-Tr(Bu$^t$)-Cys(MBzl)-AsnOBu$^t$. [11]

Das Peptid wird in Methanol gelöst und in eine Stahlbombe gefüllt. Die Stahlbombe wird gekühlt und unter Feuchtigkeitsausschluss wird flüssiges Ammoniak zugegeben. Die Bombe wird 4 Tage bei Zimmertemperatur geschüttelt. Auf diese Weise soll der γ-Benzylester unter gleichzeitiger Amidbildung abgespalten werden. Die Abspaltlösung wird im Vakuum bei 40 °C eingedampft und in Methanol aufgenommen. Es verbleibt ein unlöslicher Anteil als Rückstand.

Rohausbeute ca. 1,1 g
Unlöslicher Rückstand ca. 0,2 g

Der lösliche Anteil und der feste Rückstand werden zusammen und zur weiteren Reaktion eingesetzt.

d)4 Bildung der intrachenaren Disulfidbrücke $A^6$–$A^{11}$

Ddz-Gly-Ile-Val-Glu(OBu$^t$)-Gln-Cys-Cys(Acm)-Ala-Ser(Bu$^t$)-Val-Cys-Ser(Bu$^t$)-Leu-Tyr(Bu$^t$)-Gln-Leu-Glu(OBu$^t$)-Asn-Tyr(Bu$^t$)-Cys(MBzl)-AsnOBu$^t$. [12]

Die Reduktion der Disulfidbindungen wird mit Tributylphosphan durchgeführt; die Schliessung der Disulfidbindungen erfolgt durch Luftoxidation.

Man löst 1,3 g Insulin A-Kette [11] in 20 ml Propanol/$H_2O$ (1,2 l) und durchströmt die Lösung mit $N_2$. Dann erfolgt die Zugabe von 155,6 µl Tributylphosphan. Die Reaktionslösung lässt man 48 Stunden unter Stickstoff bei Zimmertemperatur reagieren. Danach wird die gesamte Reaktionslösung in 6 l $H_2O$, mit $NH_3$ auf pH 8 eingestellt, gegeben. Man leitet Luft während 48 Stunden durch die Lösung. Anschliessend wird im Vakuum eingeengt und durch Gelchromatographie über Sephadex LH-20® getrennt.

Man erhält 1,09 g des Materials [12], was einer Ausbeute von 84% entspricht.

Aminosäureanalyse (110 °C 6n HCl, 24 Stunden)

| | Cys CysSO$_3$H | Asp | Ser | Glu | Gly | Ala | Val | Ile | Leu | Tyr |
|---|---|---|---|---|---|---|---|---|---|---|
| berechnet: | 4 | 2 | 2 | 4 | 1 | 1 | 2 | 1 | 2 | 2 |
| gefunden: | 3,22 | 1,91 | 2,02 | 3,93 | 1,05 | 1,00a) | 1,58 | 0,67 | 2,11 | 2,21 |

a) Bezugsaminosäure

Drehwert: $[\alpha]^{25}_D = -42,0$ (C = 0,1) Methanol)

d)5 Schutzgruppenabspaltung der vereinigten Insulin A-Kette

Gly-Ile-Val-Glu-Gln-Cys-Cys(Acm)-Ala-Ser-Val-Cys-Ser-Leu-Tyr-Gln-Leu-Glu-Asn-Tyr-Cys(MBzl)-Asn [13]

[MG = 2519,90]

Die voll geschützten Insulin A-Ketten [8] und [12] werden vereinigt. Zum Peptid gibt man zwecks Schutzgruppenabspaltung konzentrierte Trifluoressigsäure und lässt eine Stunde bei Raumtemperatur stehen. Anschliessend wird die Reaktionslösung im Vakuum eingeengt. Dann wird die deblockierte Insulin A-Kette nochmals durch Gelchromatographie über Sephadex LH-20®/Methanol gereinigt. Zum Schluss wird die Insulin A-Kette gefriergetrocknet, wobei man 750 mg reine Insulin A-Kette erhält.

Voroxidation: Perameisensäure[106], 0 °C, 12 Stunden

Aminosäureanalyse: 6 n Salzsäure, 110 °C, 42 Stunden

| | CysSO$_3$H | Asp | Ser | Glu | Gly | Ala | Val | Ile | Leu | Tyr |
|---|---|---|---|---|---|---|---|---|---|---|
| berechnet: | 4 | 2 | 2 | 4 | 1 | 1 | 2 | 1 | 2 | 2 |
| gefunden: | 3,95 | 1,89 | 1,70 | 3,91 | 1,00a) | 1,08 | 1,96 | 0,69 | 2,10 | 1,67 |

a) Bezugsaminosäure

Drehwert: $[\alpha]^{25}_D = -76,2$ C = 0,5 Methanol

Das Pherogramm bei pH 1,9 1 h 1000 V ergibt ca. 3,5 cm von der Startlinie entfernt auf der Anodenseite eine breite Bande, die $Cl_{I/II}$-positiv reagiert.

e) Kombination von gereinigter synthetischer Rinderinsulin A-Kette mit natürlicher Rinderinsulin B-Kette

e)1 Abspaltung der MBzl-Schutzgruppe mit HF/Pyridin

Man löst 40,8 mg der in der obigen Weise gebildeten synthetischen Rinderinsulin A-Kette in 2 ml HF/Pyridin. Das Abspaltungsreagens dient gleichzeitig als Lösungsmittel. Als Kationenfänger werden 0,25 ml Anisol zugesetzt. Nach 60 Minuten Reaktionszeit bei Zimmertemperatur wird das an Cys 20 reduzierte Peptid mit Äther ausgefällt. Es entsteht ein klebriger weisser Niederschlag, der mit Äther mehrmals gründlich gewaschen wird.

Die deblockierte A-Kette wird dann sofort in 3 ml 30prozentiger Essigsäure gelöst.

Zuvor hat man bereits 53,4 mg reduzierte B-Kette in 2 ml 30prozentiger Essigsäure gelöst.

Die vorbereitete Lösung wird dann zur Reaktionslösung zugegeben und mit 0,1n Jodlösung titriert, bis die Jodfarbe nach ca. 4 Minuten beständig bleibt. Das überschüssige Jod wird mit Ascrobinsäure zurücktitriert, bis die Reaktionslösung farblos ist.

e)2 Reinigung des halbsynthetischen Rinderinsulins

Die Reaktionslösung wird sofort über Sephadex G-5® in 10prozentiger Essigsäure fraktioniert (Säule 1,50 × 60 cm). Die Fraktionen 3, 4, 5 und 6 werden einzeln gefriergetrocknet und elektrophoretisch mit authentischem Rinderinsulin verglichen. Die Fraktionen 3, 4 und 5 enthalten elektrophoretisch und dünnschichtchromatographisch reines Rinderinsulin. Die Rohausbeute beträgt

24,2 mg (25%). Mit dem Rohprodukt werden biologische Tests durchgeführt.

Die verschiedenen Fraktionen werden erneut einzeln in 1prozentiger Essigsäure durch eine Biogel-P-6®-Säule geführt (Säule 2 m × 0,8 cm). Die Fraktionen, die nach dieser zweiten Trennung Insulin enthalten, werden vereinigt und wiederum durch dieselbe Säule gegeben. Die Hauptfraktion enthält nun 18,2 mg Insulin (Ausbeute 20%).

Hiermit wurde erneut ein biologischer Test durchgeführt.

Voroxidation: Perameisensäure[106], 0 °C, 12 Stunden

Aminosäureanalyse: 6 n HCl, 110 °C, 42 Stunden

| | $CysSO_3H$ | Asp | Thr | Ser | Glu | Pro | Gly | Ala |
|---|---|---|---|---|---|---|---|---|
| berechnet: | 6 | 3 | 1 | 3 | 7 | 1 | 4 | 3 |
| gefunden: | 5,91 | 3,70 | 0,86 | 2,71 | 6,75 | 0,91 | 4,03 | 3,23 |

| | Val | Ile | Leu | Tyr | Phe | His | Lys | Arg |
|---|---|---|---|---|---|---|---|---|
| berechnet: | 5 | 1 | 6 | 4 | 3 | 2 | 1 | 1 |
| gefunden: | 5,12 | 0,86 | 6,00[a)] | 3,73 | 3,38 | 1,89 | 1,11 | 1,06 |

a) Bezugsaminosäure

In der Fig. 5 ist eine Mikrophotographie des Zinkkomplexes des erhaltenen voll aktiven Rinderinsulins dargestellt.

Mit Hilfe des erfindungsgemässen Verfahrens lassen sich gezielt Insulin und Insulinanaloge herstellen, die die intrachenaren und/oder interchenaren Disulfidringe in natürlicher oder unnatürlicher Anordnung enthalten und die für therapeutische Zwecke von grosser Bedeutung sind. So stellen insbesondere die antiparallelen Varianten des Insulins mit natürlicher oder unnatürlicher Anordnung des intrachenaren kleinen Disulfidrings interessante Depotformen des Insulins dar, da sie sich unter physiologischen Bedingungen ganz langsam in wirksames Insulin umwandeln. Auch das am polymeren Träger aufgebaute und gebundene Insulin mit natürlicher bzw. unnatürlicher Anordnung der intrachenaren und/oder interchenaren Disulfidringe stellt eine solche besonders langlebige, unphysiologische Insulin-Depotform dar.

Gegenstand der Erfindung sind daher auch Arzneimittel, die die mit Hilfe des erfindungsgemässen Verfahrens erhaltenen Insulinprodukte gemäss den Ansprüche 2 bis 18 mit natürlicher oder unnatürlicher Anordnung der intra- und/oder interchenaren Disulfidbrücken gegebenenfalls in Kombination mit üblichen, pharmazeutisch annehmbaren Bindemitteln, Trägermaterialien und/oder Hilfsstoffen enthalten.

**Patentansprüche**

1. Verfahren zur selektiven Bildung von drei Disulfidbrücken, gegebenenfalls in Gegenwart von einer oder mehreren schon bestehenden weiteren Disulfidbrücken, in synthetischen und/oder natürlichen Polypeptiden, dadurch gekennzeichnet, dass man wenigstens eine der beiden in eine erste Disulfidbrücke zu überführenden SH-Gruppen mit einer tert.-Butylmercaptoschutzgruppe, wenigstens eine der beiden in eine zweite Disulfidbrücke zu überführenden SH-Gruppen mit einer p-Methoxybenzylschutzgruppe und wenigstens eine der beiden in eine dritte Disulfidbrücke zu überführenden SH-Gruppen mit einer Acetamidomethylschutzgruppe maskiert, dann zuerst die tert.-Butylmercaptoschutzgruppe bzw. -gruppen mit Tributylphosphan unter Stickstoff abspaltet, mit Luft oxidiert, dann die p-Methoxybenzylschutzgruppe bzw. -gruppen mit Pyridinium-polyhydrogenfluorid (HF-Pyridin) in Gegenwart von Anisol abspaltet und anschliessend mit Jod in saurer Lösung behandelt.

2. Verfahren nach Anspruch 1, dadurch gekennzeichnet, dass man zur Herstellung von aktivem Humaninsulin eine synthetische Insulin A-Kette, die in der Position $A^7$ eine Acetamidomethylschutzgruppe und in der Position $A^{20}$ eine p-Methoxybenzylschutzgruppe aufweist, mit einer äquimolaren Menge synthetischer oder natürlicher, reduzierter Insulin B-Kette umsetzt.

3. Verfahren nach Anspruch 2, dadurch gekennzeichnet, dass man eine Insulin B-Kette einsetzt, die in der Position $B^7$ eine Acetamidomethylschutzgruppe und in der Position $B^{1a}$ eine p-Methoxybenzylschutzgruppe aufweist.

4. Verfahren nach den Ansprüchen 2 oder 3, dadurch gekennzeichnet, dass man eine synthetische $[A^6–A^{11}]$-Insulin A-Kette verwendet, deren kleiner intrachenarer Disulfidring durch reduktive Abspaltung der tert.-Butylmercaptoschutzgruppen in den Positionen $A^6$ und $A^{11}$ mit Tributylphosphan unter Stickstoff und selektiver Oxidation mit Luft gebildet worden ist.

5. Verfahren nach den Ansprüchen 2 bis 4, dadurch gekennzeichnet, dass man als natürliche Insulin B-Kette die B-Kette des Rinderinsulins verwendet, die gegebenenfalls am C-Terminus modifiziert worden ist.

6. Verfahren nach Anspruch 1, dadurch gekennzeichnet, dass man zur Herstellung von $[A^7–A^{11}$, $A^6–B^7$-Cystin]-Insulin eine synthetische $[A^7$-$A^{11}]$-Insulin A-Kette verwendet, die in der Position $A^6$ eine Acetamidomethylschutzgruppe und in der

Position $A^{20}$ eine p-Methoxybenzylschutzgruppe aufweist.

7. Verfahren nach Anspruch 1, dadurch gekennzeichnet, dass man zur Herstellung von [$A^6$–$A^7$, $A^{11}$–$B^7$-Cystin]-Insulin eine synthetische [$A^6$–$A^7$]-Insulin A-Kette verwendet, die in der Position $A^{11}$ eine Acetamidomethylschutzgruppe und in der Position $A^{20}$ eine p-Methoxybenzylschutzgruppe aufweist.

8. Verfahren nach den Ansprüchen 1 bis 7, dadurch gekennzeichnet, dass man die Disulfidbrückenbildung durch Oxidation mit Jod in 30%iger Essigsäure bewirkt.

9. Verfahren nach den Ansprüchen 1 bis 8, dadurch gekennzeichnet, dass man bei der Abspaltung der p-Methoxybenzylschutzgruppe bzw. -gruppen in Pyridinium-polyhydrogenfluorid (HF/Pyridin) als Lösungsmittel und Reagens arbeitet.

10. Verfahren nach Anspruch 1, dadurch gekennzeichnet, dass man zur Herstellung von antiparallelem [$A^6$–$A^{11}$, $A^7$–$B^{19}$, $A^{20}$–$B^7$-Cystin]-Insulin eine natürliche oder synthetische [$A^6$–$A^{11}$]-Insulin A-Kette, die in der Position $A^7$ eine Acetamidomethylschutzgruppe und in der Position $A^{20}$ eine p-Methoxybenzylschutzgruppe aufweist, mit einer natürlichen oder synthetischen Insulin B-Kette umsetzt, die in reduzierter Form vorliegt, oder bevorzugter in der Position $B^{19}$ eine Acetamidomethylschutzgruppe und in der Position $B^7$ eine p-Methoxybenzylschutzgruppe aufweist.

11. Verfahren nach Anspruch 1, dadurch gekennzeichnet, dass man zur Herstellung von antiparallelem [$A^6$–$A^{11}$, $A^7$–$B^{19}$, $A^{20}$–$B^7$-Cystin]-Insulin eine natürliche oder synthetische [$A^6$–$A^{11}$]-Insulin A-Kette, die in der Position $A^{20}$ eine Acetamidomethylschutzgruppe und in der Position $A^7$ eine p-Methoxybenzylschutzgruppe aufweist, mit einer natürlichen oder synthetischen Insulin B-Kette umsetzt, die in reduzierter Form vorliegt, oder bevorzugter in der Position $B^7$ eine Acetamidomethylschutzgruppe und in der Position $B^{19}$ eine p-Methoxybenzylschutzgruppe aufweist.

12. Verfahren nach Anspruch 1, dadurch gekennzeichnet, dass man zur Herstellung von antiparallelem [$A^7$–$A^{11}$, $A^6$–$B^{19}$, $A^{20}$–$B^7$-Cystin]-Insulin eine synthetische [$A^7$–$A^{11}$]-Insulin A-Kette, die in der Position $A^6$ eine Acetamidomethylschutzgruppe und in der Position $A^{20}$ eine p-Methoxybenzylschutzgruppe aufweist, mit einer natürlichen oder synthetischen Insulin B-Kette umsetzt, die in reduzierter Form vorliegt, oder bevorzugter in der Position $B^{19}$ eine Acetamidomethylschutzgruppe und in der Position $B^7$ eine p-Methoxybenzylschutzgruppe aufweist.

13. Verfahren nach Anspruch 1, dadurch gekennzeichnet, dass man zur Herstellung von antiparallelem [$A^7$–$A^{11}$, $A^6$–$B^{19}$, $A^{20}$–$B^7$-Cystin]-Insulin eine synthetische [$A^7$–$A^{11}$]-Insulin A-Kette, die in der Position $A^{20}$ eine Acetamidomethylschutzgruppe und in der Position $A^6$ eine p-Methoxybenzylschutzgruppe aufweist, mit einer natürlichen oder synthetischen Insulin B-Kette umsetzt, die in reduzierter Form vorliegt, oder bevorzugter in der Position $B^7$ eine Acetamidomethylschutzgruppe und in der Position $B^{19}$ eine p-Methoxybenzylschutzgruppe aufweist.

14. Verfahren nach Anspruch 1, dadurch gekennzeichnet, dass man zur Herstellung von antiparallelem [$A^6$–$A^7$, $A^{11}$–$B^{19}$, $A^{20}$–$B^7$-Cystin]-Insulin eine synthetische [$A^6$–$A^7$]-Insulin A-Kette, die in der Position $A^{11}$ eine Acetamidomethylschutzgruppe und in der Position $A^{20}$ eine p-Methoxybenzylschutzgruppe aufweist, mit einer natürlichen oder synthetischen Insulin B-Kette umsetzt, die in reduzierter Form vorliegt, oder bevorzugter in der Position $B^{19}$ eine Acetamidomethylschutzgruppe und in der Position $B^7$ eine p-Methoxybenzylschutzgruppe aufweist.

15. Verfahren nach Anspruch 1, dadurch gekennzeichnet, dass man zur Herstellung von antiparallelem [$A^6$–$A^7$, $A^{11}$–$B^{19}$, $A^{20}$–$B^7$-Cystin]-Insulin eine synthetische [$A^6$–$A^7$]-Insulin A-Kette, die in der Position $A^{20}$ eine Acetamidomethylschutzgruppe und in der Position $A^{11}$ eine p-Methoxybenzylschutzgruppe aufweist, mit einer natürlichen oder synthetischen Insulin B-Kette umsetzt, die in reduzierter Form vorliegt, oder bevorzugter in der Position $B^7$ eine Acetamidomethylschutzgruppe und in der Position $B^{19}$ eine p-Methoxybenzylschutzgruppe aufweist.

16. Verfahren nach einem der vorhergehenden Ansprüche, dadurch gekennzeichnet, dass man eine natürliche oder synthetische Insulin A-Kette mit einem intrachenaren Disulfidring in der $A^6$–$A^{11}$-Position, in der $A^7$–$A^{11}$-Position oder in der $A^6$–$A^7$-Position an einem polymeren Träger parallel oder antiparallel mit einer natürlichen oder synthetischen Insulin B-Kette, die in reduzierter Form vorliegt, oder Acetamidomethylschutzgruppen und p-Methoxybenzylschutzgruppen in den in den Ansprüchen 10 bis 15 definierten Positionen aufweist, Disulfid-verbrückt.

17. Verfahren nach Anspruch 16, dadurch gekennzeichnet, dass man als polymeren Träger mit Divinylbenzol vernetztes Polystyrol verwendet.

18. Verfahren nach Anspruch 17, dadurch gekennzeichnet, dass man ein Polystyrol verwendet, das mit 0,1 bis 0,8%, vorzugsweise mit 0,5% Divinylbenzol vernetzt ist.

19. Arzneimittel, dadurch gekennzeichnet, dass sie mindestens eines der gemäss den Ansprüchen 2 bis 18 hergestellten Insulinprodukte mit natürlicher oder unnatürlicher Anordnung der intra- und/oder interchenaren Disulfidbrücken, gegebenenfalls in Kombination mit üblichen, pharmazeutisch annehmbaren Bindemitteln, Trägermaterialien und/oder Hilfsstoffen enthalten.

**Claims**

1. Process for the selective formation of three disulphide bridges, optionally in the presence of one or more already existing further disulphide bridges, in synthetic and/or natural polypeptides, characterised in that one masks at least one of the two SH groups to be converted into a first disulphide bridge with a tert.-butylmercapto protective

group, at least one of the two SH groups to be converted into a second disulphide group with a p-methoxybenzyl protective group and at least one of the two SH groups to be converted into a third disulphide bridge with an acetamidomethyl protective group, then splits off first the tert.-butylmercapto protective group or groups with tributyl phosphane under nitrogen, oxidises with air, then splits off the p-methoxybenzyl protective group or groups with pyridinium polyhydrogen fluoride (HF-pyridine) in the presence of anisole and finally treats with iodine in acid solution.

2. Process according to claim 1, characterised in that, for the preparation of active human insulin, one reacts a synthetic insulin A chain, which has an acetamidomethyl protective group in the position $A^7$ and a p-methoxybenzyl protective group in position $A^{20}$, with an equimolar amount of synthetic or natural, reduced insulin B chain.

3. Process according to claim 2, characterised in that one reacts an insulin B chain which has an acetamidomethyl protective group in position $B^7$ and a p-methoxybenzyl protective group on position $B^{19}$.

4. Process according to claim 2 or 3, characterised in that one uses a synthetic [$A^6$–$A^{11}$]-insulin A chain the smaller intra-chain disulphide ring of which has been formed by reductive splitting off of the tert.-butylmercapto protective group in positions $A^6$ and $A^{11}$ with tributyl phosphane under nitrogen and selective oxidation with air.

5. Process according to claims 2 to 4, characterised in that, as natural insulin B chain, one uses the B chain of bovine insulin which has optionally been modified on the C terminus.

6. Process according to claim 1, characterised in that, for the preparation of [$A^7$–$A^{11}$, $A^6$–$B^7$-cystine]-insulin, one uses a synthetic [$A^7$–$A^{11}$]-insulin A chain which has an acetamidomethyl protective group in position $A^6$ and a p-methoxybenzyl protective group in position $A^{20}$.

7. Process according to claim 1, characterised in that, for the preparation of [$A^6$–$A^7$, $A^{11}$–$B^7$-cystine]-insulin, one uses a synthetic [$A^6$–$A^7$]-insulin A chain which has an acetamidomethyl protective group in position $A^{11}$ and a p-methoxybenzyl protective group in position $A^{20}$.

8. Process according to claims 1 to 7, characterised in that one brings about the disulphide bridge formation by oxidation with iodine in 3% acetic acid.

9. Process according to claims 1 to 8, characterised in that, in the case of the splitting off of the p-methoxybenzyl protective group or groups, one works in pyridinium polyhydrogen fluoride (HF/pyridine) as solvent and reagent.

10. Process according to claim 1, characterised in that, for the preparation of anti-parallel [$A^6$–$A^{11}$, $A^7$–$B^{19}$, $A^{20}$–$B^7$-cystine]-insulin, one reacts a natural or synthetic [$A^6$–$A^{11}$]-insulin A chain, which has an acetamidomethyl protective group in position $A^7$ and a p-methoxybenzyl protective group in position $A^{20}$, with a natural or synthetic insulin B chain, which is present in reduced form or, more preferably, has an acetamidomethyl protective group in position $B^{19}$ and a p-methoxybenzyl protective group in position $B^7$.

11. Process according to claim 1, characterised in that, for the preparation of anti-parallel [$A^6$–$A^{11}$, $A^7$–$B^{19}$, $A^{20}$–$B^7$-cystine]-insulin, one reacts a natural or synthetic [$A^6$–$A^{11}$]-insulin A chain, which has an acetamidomethyl protective group in position $A^{20}$ and a p-methoxybenzyl protective group in position $A^7$, with a natural or synthetic insulin B chain, which is present in reduced form or, more preferably, has an acetamidomethyl protective group in position $B^7$ and a p-methoxybenzyl protective group in position $B^{19}$.

12. Process according to claim 1, characterised in that, for the preparation of anti-parallel [$A^7$–$A^{11}$, $A^6$–$B^{19}$, $A^{20}$–$B^7$-cystine]-insulin, one reacts a synthetic [$A^7$–$A^{11}$]-insulin A chain, which has an acetamidomethyl protective group in position $A^6$ and a p-methoxybenzyl protective group in position $A^{20}$, with a natural or synthetic insulin B chain which is present in reduced form or, more preferably, has an acetamidomethyl protective group in position $B^{19}$ and a p-methoxybenzyl protective group in position $B^7$.

13. Process according to claim 1, characterised in that, for the preparation of anti-parallel [$A^7$–$A^{11}$, $A^6$–$B^{19}$, $A^{20}$–$B^7$-cystine]-insulin, one reacts a synthetic [$A^7$–$A^{11}$]-insulin A chain, which has an acetamidomethyl protective group in position $A^{20}$ and a p-methoxybenzyl protective group in position $A^6$, with a natural or synthetic insulin B chain which is present in reduced form or, more preferably, has an acetamidomethyl protective group in position $B^7$ and a p-methoxybenzyl protective group in position $B^{19}$.

14. Process according to claim 1, characterised in that, for the preparation of anti-parallel [$A^6$–$A^7$, $A^{11}$–$B^{19}$, $A^{20}$–$B^7$-cystine]-insulin, one reacts a synthetic [$A^6$–$A^7$]-insulin A chain, which has an acetamidomethyl protective group in position $A^{11}$ and a p-methoxybenzyl protective group in position $A^{20}$, with a natural or synthetic insulin B chain which is present in reduced form or, more preferably, has an acetamidomethyl protective group in position $B^{19}$ and a p-methoxybenzyl protective group in position $B^7$.

15. Process according to claim 1, characterised in that, for the preparation of anti-parallel [$A^6$–$A^7$, $A^{11}$–$B^{19}$, $A^{20}$–$B^7$-cystine]-insulin, one reacts a synthetic [$A^6$–$A^7$]-insulin A chain, which has an acetamidomethyl protective group in position $A^{20}$ and a p-methoxybenzyl protective group in position $A^{11}$, with a natural or synthetic insulin B chain which is present in reduced form or, more preferably, has an acetamidomethyl protective group in position $B^7$ and a p-methoxybenzyl protective group in position $B^{19}$.

16. Process according to one of the preceding claims, characterised in that one disulphide bridges a natural or synthetic insulin A chain with an intrachain disulphide ring in the $A^6$–$A^{11}$ position, in the $A^7$–$A^{11}$ position or in the $A^6$–$A^7$ position on a polymeric carrier parallel or anti-parallel with a natural or synthetic insulin B chain which is present in reduced form or has acetamidomethyl protective groups and p-methoxybenzyl protective groups in the positions defined in claims 10 to 15.

17. Process according to claim 16, characterised in that one uses polystyrene cross-linked with divinylbenzene as polymeric carrier.

18. Process according to claim 17, characterised in that one uses a polystyrene gel which is cross-linked with 0.1 to 0.8%, preferably with 0.5% divinylbenzene.

19. Pharmaceutical, characterised in that it contains at least one of the insulin products prepared according to claims 2 to 18 with natural or non-natural arrangement of the intra- and/or interchain disulphide bridges, optionally in combination with conventional pharmaceutically acceptable binding agents, carrier materials and/or adjuvants.

**Revendications**

1. Procédé pour la formation sélective de trois ponts disulfure, éventuellement en présence d'un ou de plusieurs autres ponts disulfure déjà existants, dans des polypeptides synthétiques et/ou naturels, caractérisé en ce que l'un au moins des deux groupes SH devant former un premier pont disulfure avec un groupe de protection tert-butylmercapto, au moins l'un des deux groupes SH devant former un deuxième pont disulfure avec un groupe de protection para-méthoxybenzyle, et au moins l'un des deux groupes SH devant former un troisième pont disulfure avec un groupe de protection acétamidométhyle, puis que l'on détache d'abord le ou les groupes de protection tert-butylmercapto avec du tributylphosphane sous azote, qu'on oxyde avec de l'air, puis que l'on détache le ou les groupes de protection para-méthoxybenzyle avec du pyridinium-polyfluorure d'hydrogène (HF-pyridine) en présence d'anisole et qu'ensuite, on traite avec de l'iode en solution acide.

2. Procédé selon la revendication 1, caractérisé en ce que, pour la préparation d'insuline humaine active, on fait réagir une chaîne A d'insuline de synthèse, qui présente en position $A^7$ un groupe de protection acétamidométhyle et en position $A^{20}$ un groupe de protection para-méthoxybenzyle, avec une quantité équimolaire de chaîne B d'insuline réduite de synthèse ou naturelle.

3. Procédé selon la revendication 2, caractérisé en ce qu'on utilise une chaîne B d'insuline qui présente en position $B^7$ un groupe de protection acétamidométhyle et en position $B^{19}$ un groupe de protection para-méthoxybenzyle.

4. Procédé selon l'une des revendications 2 ou 3, caractérisé en ce qu'on utilise une chaîne A de [$A^6$–$A^{11}$]-insuline de synthèse, dont le petit cycle disulfure intra-chaînes a été formé par un détachement réducteur des groupes tert-butylmercapto de protection aux positions $A^6$ et $A^{11}$ avec du tributylphosphane sous azote et oxydation sélective avec de l'air.

5. Procédé selon l'une des revendications 2 à 4, caractérisé en ce que l'on utilise à titre de chaîne B d'insuline naturelle la chaîne B d'insuline bovine dont éventuellement le C-terminal a été modifié.

6. Procédé selon la revendication 1, caractérisé en ce que l'on utilise pour la fabrication de la [$A^7$–$A^{11}$, $A^6$–$B^7$-cystine]-insuline, une chaîne A de [$A^7$–$A^{11}$]-insuline de synthèse, qui présente en position $A^6$ un groupe de protection acétamidométhyle et en position $A^{20}$ un groupe de protection para-méthoxybenzyle.

7. Procédé selon la revendication 1, caractérisé en ce que pour la fabrication de [$A^6$–$A^7$, $A^{11}$–$B^7$-cystine]-insuline, on utilise une chaîne A de [$A^6$–$A^7$]-insuline de synthèse, qui présente en position $A^{11}$ un groupe de protection acétamidométhyle et en position $A^{20}$ un groupe de protection para-méthoxybenzyle.

8. Procédé selon l'une des revendications 1 à 7, caractérisé en ce que l'on effectue la formation des ponts disulfure par oxydation avec de l'iode dans de l'acide acétique à 30%.

9. Procédé selon l'une des revendications 1 à 8, caractérisé en ce que, pour le détachement du ou des groupes de protection para-méthoxybenzyle, on travaille avec du pyridinium-polyfluorure d'hydrogène (HF/pyridine) servant de solvant et de réactif.

10. Procédé selon la revendication 1, caractérisé en ce que, pour la fabrication de la [$A^6$–$A^{11}$, $A^7$–$B^{19}$, $A^{20}$–$B^7$-cystine]-insuline antiparallèle, on fait réagir une chaîne A de [$A^6$–$A^{11}$]-insuline synthétique ou naturelle qui porte en position $A^7$ un groupe de protection acétamidométhyle et en position $A^{20}$ un groupe de protection para-méthoxybenzyle, avec une chaîne B d'insuline naturelle ou synthétique, qui se trouve sous forme réduite, ou de préférence présente à la position $B^{19}$ un groupe de protection acétamidométhyle et à la position $B^7$ un groupe de protection para-méthoxybenzyle.

11. Procédé selon la revendication 1, caractérisé en ce que, pour la fabrication de la [$A^6$–$A^{11}$, $A^7$–$B^{19}$, $A^{20}$–$B^7$-cystine]-insuline antiparallèle, on fait réagir une chaîne A de [$A^6$–$A^{11}$]-insuline naturelle ou synthétique, qui présente à la position $A^{20}$ un groupe de protection acétamidométhyle et à la position $A^7$ un groupe de protection para-méthoxybenzyle, avec une chaîne B d'insuline naturelle ou synthétique, qui se trouve sous forme réduite ou de préférence présente à la position $B^7$ un groupe de protection acétamidométhyle et à la position $B^{19}$ un groupe de protection para-méthoxybenzyle.

12. Procédé selon la revendication 1, caractérisé en ce que, pour la fabrication de la [$A^7$–$A^{11}$, $A^6$–$B^{19}$, $A^{20}$–$B^7$-cystine]-insuline antiparallèle, on fait réagir une chaîne A de [$A^7$–$A^{11}$]-insuline synthétique, qui présente à la position $A^6$ un groupe de protection acétamidométhyle et à la position $A^{20}$ un groupe de protection para-méthoxybenzyle, avec une chaîne B d'insuline naturelle ou de synthèse qui se trouve sous forme réduite ou de préférence présente à la position $B^{19}$ un groupe de protection acétamidométhyle et à la position $B^7$ un groupe de protection para-méthoxybenzyle.

13. Procédé selon la revendication 1, caractérisé en ce que, pour la fabrication de la [$A^7$–$A^{11}$,

$A^6$–$B^{19}$, $A^{20}$–$B^7$-cystine]-insuline antiparallèle, on fait réagir une chaîne A de [$A^7$–$A^{11}$]-insuline de synthèse, qui présente à la position $A^{20}$ un groupe de protection acétamidométhyle et à la position $A^6$ un groupe de protection para-méthoxybenzyle, avec une chaîne B d'insuline naturelle ou de synthèse, qui se trouve sous forme réduite ou de préférence présente à la position $B^7$ un groupe de protection acétamidométhyle et à la position $B^{19}$ un groupe de protection para-méthoxybenzyle.

14. Procédé selon la revendication 1, caractérisé en ce que, pour la fabrication de la [$A^6$–$A^7$, $A^{11}$–$B^{19}$, $A^{20}$–$B^7$-cystine]-insuline antiparallèle, on fait réagir une chaîne A de [$A^6$–$A^7$]-insuline de synthèse qui présente à la position $A^{11}$ un groupe de protection acétamidométhyle et à la position $A^{20}$ un groupe de protection para-méthoxybenzyle, avec une chaîne B d'insuline naturelle ou de synthèse qui se trouve sous forme réduite ou de préférence présente à la position $B^{19}$ un groupe de protection acétamidométhyle et à la position $B^7$ un groupe de protection para-méthoxybenzyle.

15. Procédé selon la revendication 1, caractérisé en ce que, pour la fabrication de la [$A^6$–$A^7$, $A^{11}$–$B^{19}$, $A^{20}$–$B^7$-cystine]-insuline antiparallèle, on fait réagir une chaîne A de [$A^6$–$A^7$]-insuline de synthèse, qui présente à la position $A^{20}$ un groupe de protection acétamidométhyle et à la position $A^{11}$ un groupe de protection para-méthoxybenzyle, avec une chaîne B d'insuline naturelle ou de synthèse, qui se trouve sous forme réduite ou de préférence présente à la position $B^7$ un groupe de protection acétamidométhyle et à la position $B^{19}$ un groupe de protection para-méthoxybenzyle.

16. Procédé selon l'une quelconque des revendications 1 à 15, caractérisé en ce que l'on réunit par des ponts disulfure, une chaîne A d'insuline naturelle ou synthétique présentant un cycle disulfure intra-chaînes en position $A^6$–$A^{11}$, en position $A^7$–$A^{11}$ ou en position $A^6$–$A^7$ sur un support polymère parallèle ou antiparallèle, avec une chaîne B d'insuline naturelle ou synthétique, qui se trouve sous forme réduite ou présente des groupes de protection acétamidométhyle ou des groupes de protection para-méthoxybenzyle, aux positions définies selon les revendications 10 à 15.

17. Procédé selon la revendication 16, caractérisé en ce que l'on utilise comme support polymère, du polystyrène réticulé par du divinylbenzène.

18. Procédé selon la revendication 17, caractérisé en ce que l'on utilise un gel de polystyrène qui est réticulé avec 0,1 à 0,8%, et de préférence avec 0,5% de divinylbenzène.

19. Médicament, caractérisé en ce qu'il renferme au moins un produit de l'insuline fabriqué selon l'une des revendications 2 à 18, présentant un arrangement naturel ou non naturel des ponts disulfure intra- et/ou inter-chaînes, éventuellement en association avec des liants, supports et/ou auxiliaires usuels acceptables du point de vue pharmaceutique.

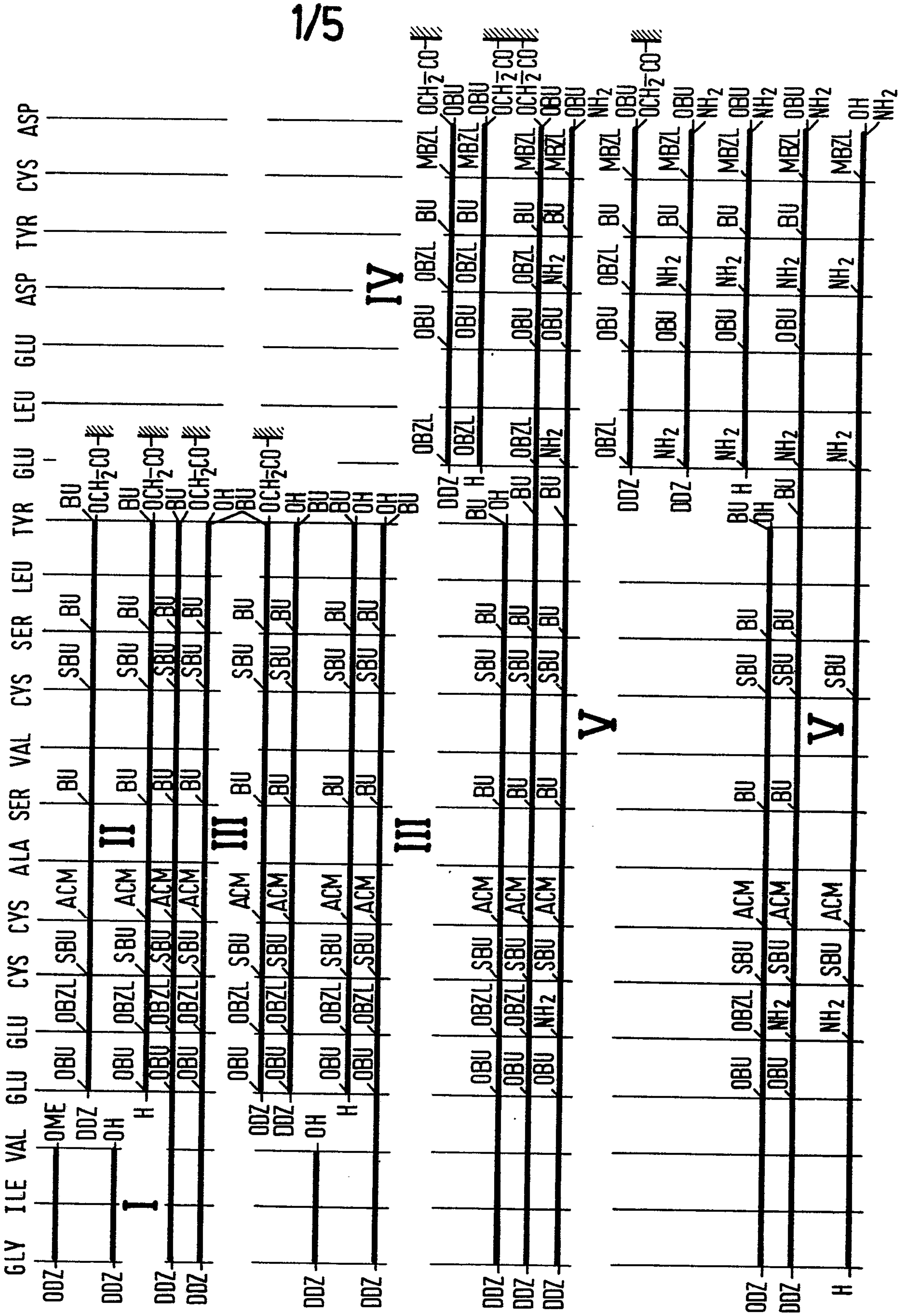
GLY ILE VAL GLU GLU CYS CYS ALA SER VAL CYS SER LEU TYR GLU LEU GLU ASP TYR CYS ASP
I
II
III
IV
V

FIG.1

2/5

FIG.2

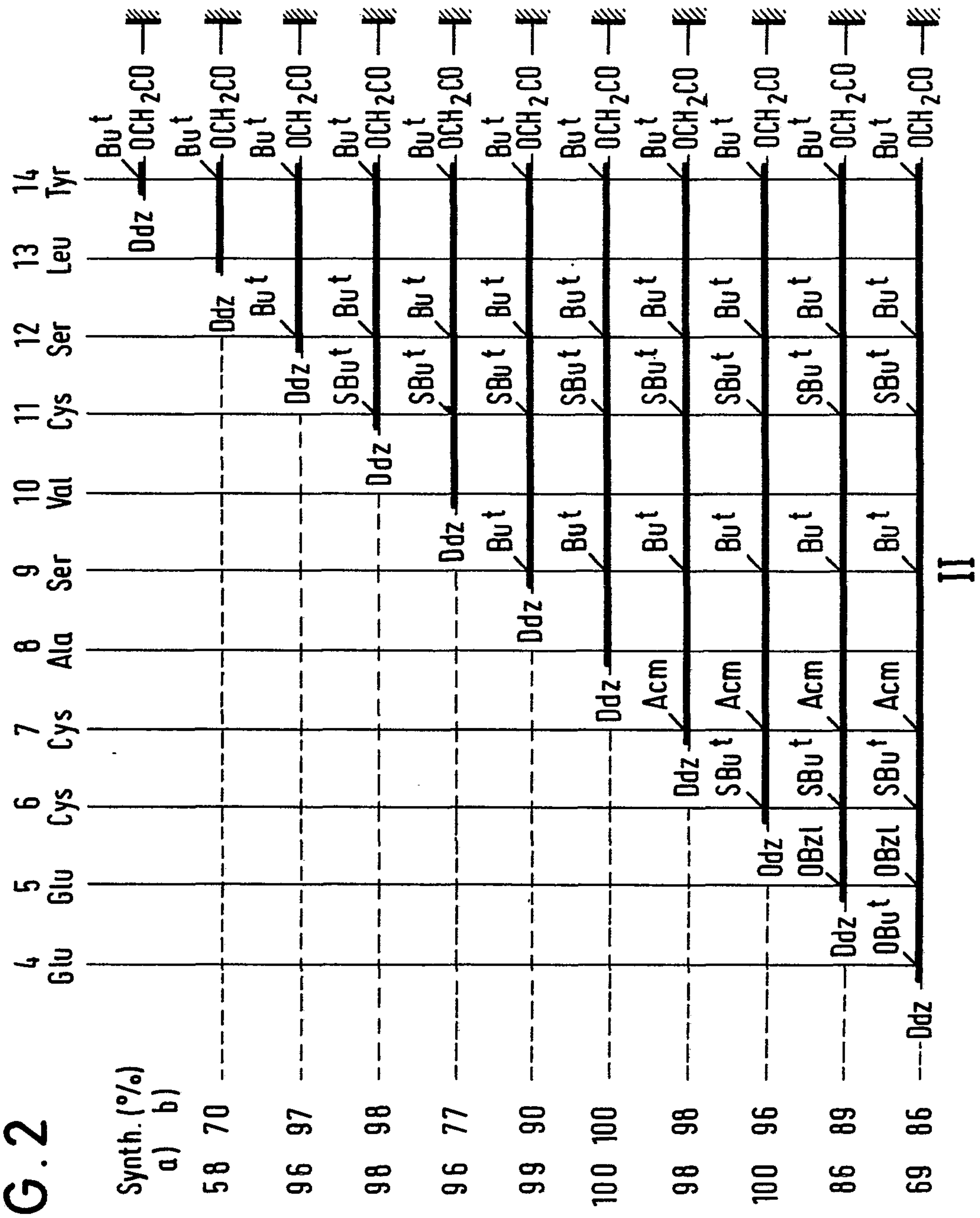
Synth. (%)
a) b)
58 70
96 97
98 98
96 77
99 90
100 100
98 98
100 96
86 89
69 86
4 Glu
5 Glu
6 Cys
7 Cys
8 Ala
9 Ser
10 Val
11 Cys
12 Ser
13 Leu
14 Tyr
Ddz
Bu^t
$OCH_2CO$
SBu^t
Acm
OBzl
OBu^t
II

3/5

FIG.3

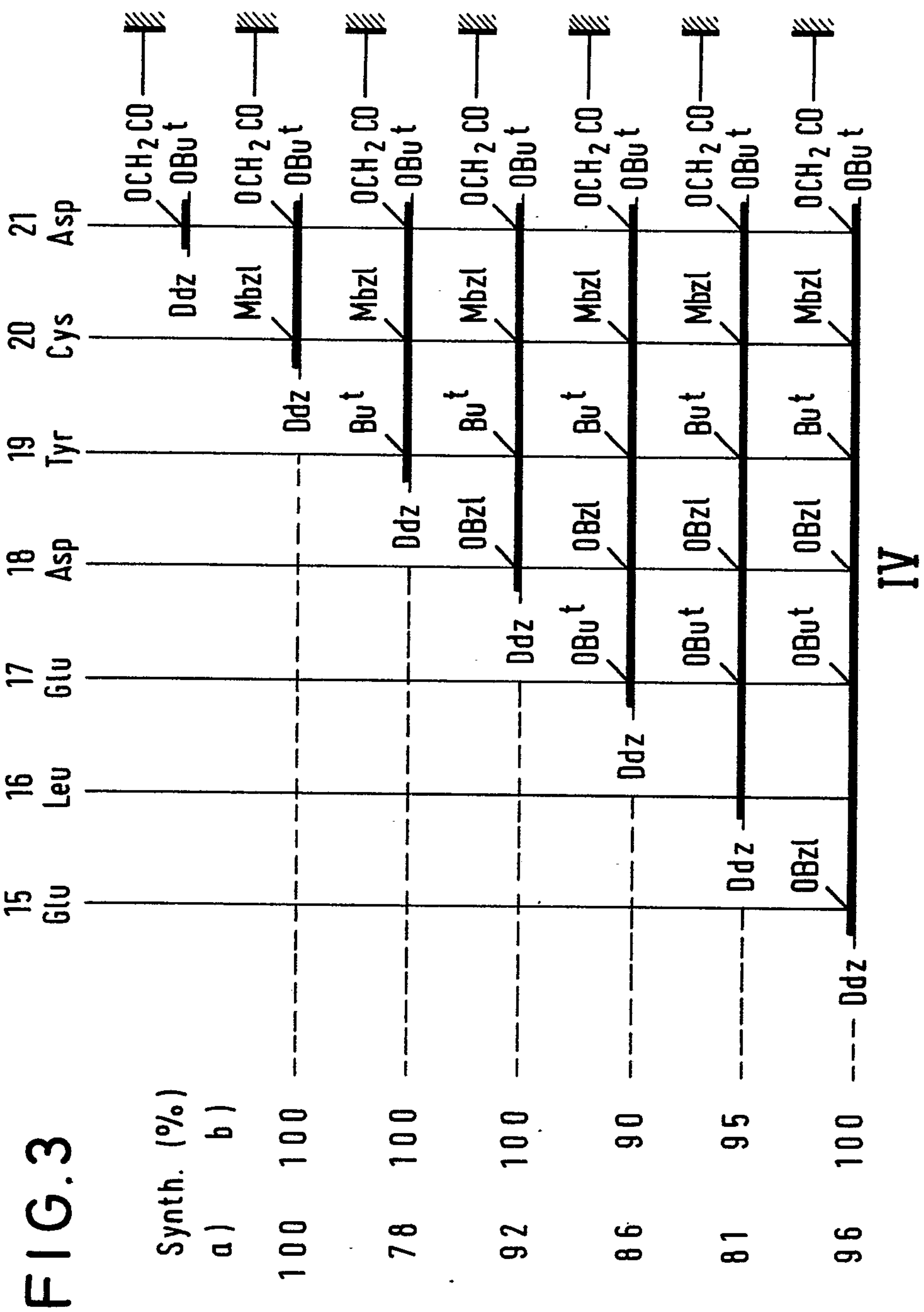

Synth. (%)
a) b)
100 100
78 100
92 100
86 90
81 95
96 100
15 Glu
16 Leu
17 Glu
18 Asp
19 Tyr
20 Cys
21 Asp
Ddz
OBzl
OBu t
Bu t
Mbzl
$OCH_2CO$
IV

4/5

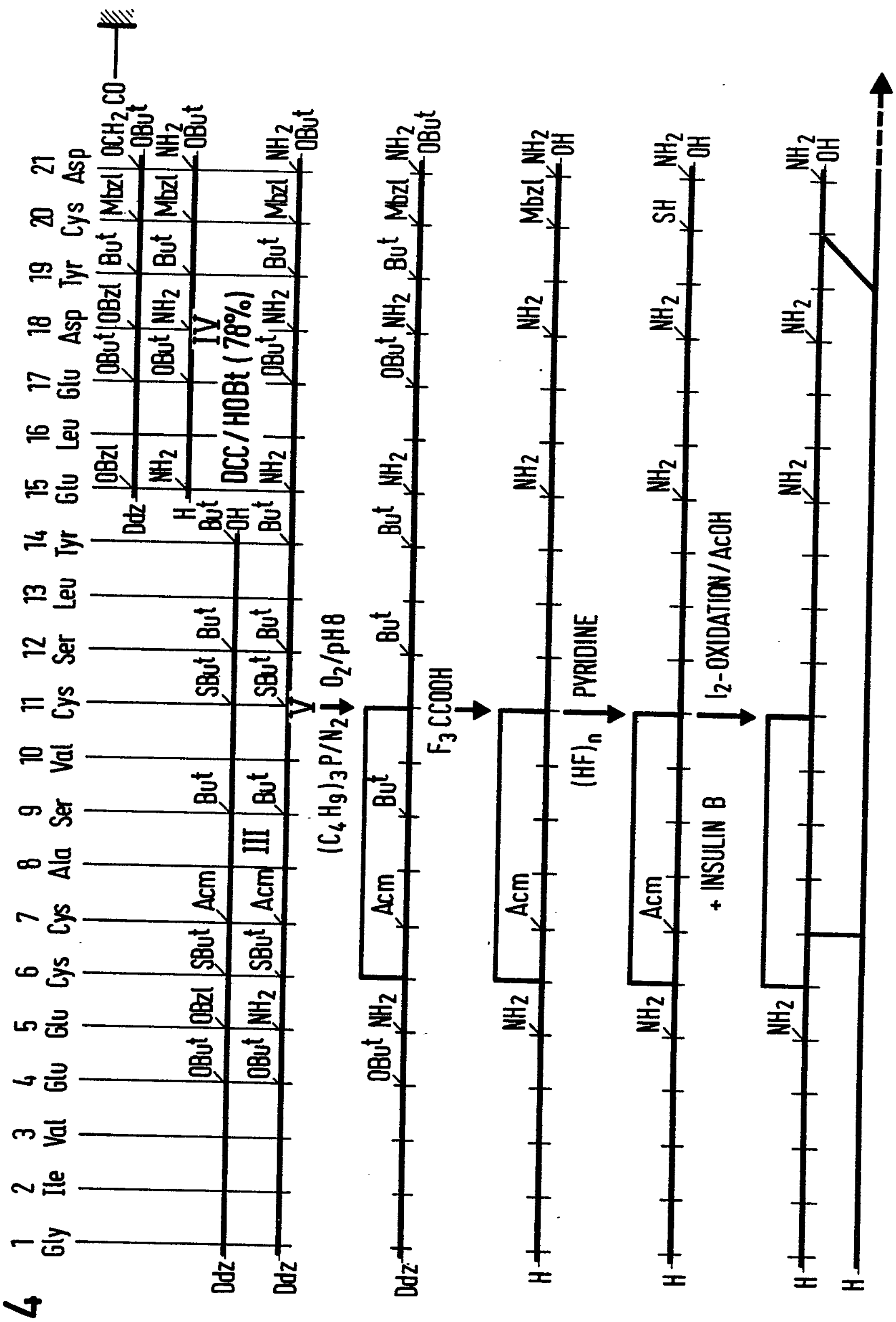
1 Gly
2 Ile
3 Val
4 Glu
5 Glu
6 Cys
7 Cys
8 Ala
9 Ser
10 Val
11 Cys
12 Ser
13 Leu
14 Tyr
15 Glu
16 Leu
17 Glu
18 Asp
19 Tyr
20 Cys
21 Asp
OCH2 CO
III
IV
DCC/HOBt (78%)
V
(C4H9)3P/N2
O2/pH8
F3 CCOOH
(HF)n
PYRIDINE
I2-OXIDATION/AcOH
+ INSULIN B

FIG. 4

FIG. 5